# EUROPÄISCHE PATENTSCHRIFT

(11) **EP 2 678 054 B1**
(45) Veröffentlichungstag und Bekanntmachung des Hinweises auf die Patenterteilung: **08.04.2015**
(21) Anmeldenummer: 12714924.3
(22) Anmeldetag: 23.02.2012
(51) Int. Cl.: A61M 5/34

(54) **INJEKTIONSSPRITZENKOPF, AUSPRESSEINHEIT SOWIE DARAUS GEBILDETE INJEKTIONSSPRITZE**
HEAD OF AN INJECTION SYRINGE, INJECTION ASSEMBLY AND INJECTION SYRINGE
TETE DE SERINGUE D'INJECTION, DISPOSITIF D'INJECTION ET SERINGUE D'INJECTION

(30) Priorität: 23.02.2011 AT 1072011; 12.05.2011 AT 6802011
(43) Veröffentlichungstag der Anmeldung: 01.01.2014
(73) Patentinhaber: Pharma Consult Ges.m.b.H. & Co Nfg KG, A-1210 Wien (AT)
(72) Erfinder: PICKHARD, Ewald, 2203 Großebersdorf (AT); SCHWIRTZ, Andreas, 1090 Wien (AT)
(74) Vertreter: Burger, Hannes
(86) Internationale Anmeldenummer: PCT/AT2012/050024
(87) Internationale Veröffentlichungsnummer: WO 2012/113008

(56) Entgegenhaltungen:
- EP-A1- 1 232 763
- WO-A1-2007/112470
- WO-A1-2009/097634
- AT-B- 404 430
- US-B1- 6 613 016

## Beschreibung

Die Erfindung betrifft eine Injektionsspritze umfassend einen Spritzenzylinder zur Aufnahme eines abzugebenden Medikaments, eine am distalen Ende des Spritzenzylinders angeordnete Auspresseinheit, einen am proximalen Ende des Spritzenzylinders angeordneten Injektionsspritzenkopf mit einer in einer Führungshülse angeordneten und darin gleitverschieblichen Nadeleinheit mit einer Kanüle und einem Kanülenhalter, und einem Triebling, durch den die Nadeleinheit axial relativ zum Spritzenzylinder von einer Ruhestellung hin zu einer Aktivierungsstellung bewegbar ist, und einem am distalen Ende der Führungshülse angeordneten Dichtelement, welches in der Ruhestellung der Nadeleinheit von der Kanüle undurchstochen und in der Aktivierungsstellung der Nadeleinheit von der Kanüle durchstochen ist.

In der vorliegenden Beschreibung werden die Richtungen "proximal" und "distal" von der Seite des Patienten betrachtet definiert. Damit ist die proximale Seite bei den nachfolgend beschriebenen Bauteilen stets dem Patienten zugewendet und die distale Seite stets von diesem abgewendet. Injektionsspritzen, insbesondere Einwegspritzen, sind bekannte Mittel zum Verabreichen von Medikamenten. Bei Einwegspritzen, die oftmals vom Patienten selbst angewendet werden, wird die Kanüle erst unmittelbar vor der Anwendung durch eine Drehbewegung mit dem Spritzenzylinder verbunden.

Aus der AT 404 430 B ist ein Injektionsspritzenkopf bekannt, bei welchem die Kanüle wahlweise durch eine Links- oder durch eine Rechtsdrehung eine Dichtscheibe am Ausgang des Spritzenzylinders durchsticht, sodass der Patient zuvor keiner Anleitung bedarf, in welcher Richtung zu drehen ist, oder ob eine Drehrichtung einer sicheren Anwendung der Injektionsspritze entgegensteht. Dafür ist gemäß der Lehre der AT 404 430 B ein Kanülenträger in eine Kanülenträgerführung eingesetzt, wobei der Kanülenträger eine sich zum proximalen Ende verzweigende, Y-förmige Führungsnut aufweist, in die ein Führungszapfen in der Kanülenträgerführung eingreift. Beim Drehen des Kannlenträgers durchläuft der Führungszapfen entweder den linken oder den rechten Schenkel der Nut, wodurch sich der Kanülenträger in einer Rechts- oder Linksdrehung in Richtung Dichtscheibe bewegt. Der Nachteil dieser Lösung besteht darin, dass der Führungszapfen an der Innenseite der Kanülenträgerführung angeordnet sein muss, was in der Herstellung aufwendig und mit hohen Ausschussraten verbunden ist. Ein weiterer Nachteil besteht darin, dass ein Herausführen des Kanülenträgers aus der Endposition weiterhin möglich ist, der Anwender also absichtlich oder unabsichtlich die Verbindung von Kanüle und angestochener Dichtscheibe wieder lösen kann. Weiters ist es nachteilig, dass sich das zugeschärfte distale Ende der Kanüle in die Dichtscheibe hineindreht und beim Einstechen Abriebpartikel aus dem Material der Dichtscheibe erzeugen kann, die dann in das flüssige Medikament gelangen. Weiters kann die benutzte Kanüle nach dem Gebrauch nur durch erneutes Aufsetzen eines Teils der Schutzkappe wieder geschützt werden, was mit Stichverletzungen verbunden sein kann.

Aus der US 5 250 037 A bzw. der DE 693 19 702 T2 ist ein Injektionsspritzenkopf der einleitend angegebenen Art bekannt, bei welcher ein Triebling eine Nadeleinheit vor der Injektion mit dem Spritzenzylinder verbindet. Nachteilig ist hier, dass der Triebling gleichzeitig die Schutzkappe der Injektionsspritze ist und nach der Betätigung von ihr entfernt wird. In der Folge bleibt die Nadeleinheit nur locker und unarretiert mit dem Spritzenzylinder während des Injektionsvorganges verbunden. Auch hier kann die benutzte Kanüle nach dem Gebrauch nur durch erneutes Aufsetzen der Schutzkappe wieder geschützt werden, was mit Stichverletzungen verbunden sein kann.

Eine andere Injektionsspritze ist aus der WO 2009/097634 A1 bekannt geworden. Dabei umfasst diese Injektionsspritze einen von einer Schutzkappe im Ausgangszustand abgedeckten Injektionsspritzenkopf, welcher seinerseits eine in einem Kanülenhalter gehaltene Kanüle, eine den Kanülenhalter samt der Kanüle aufnelunenden Führungshülse sowie einen Antriebsteil für die Verstellung der Kanüle samt dem Kanülenhalter aufweist. An dem dem Spritzenzylinder zugewendeten Ende ist an der Führungshülse ein Dichteinsatz aufgeschnappt. Zur Umsetzung der Drehbewegung ausgehend von der Schutzkappenspitze auf den Antriebsteil und in weiterer Folge auf den Kanülenhalter ist der Antriebsteil an seiner Außenseite mit einer Kulissenbahn versehen, welche mit einem Führungszapfen an der Innenseite der Führungshülse zusammenwirkt. Auch hier besteht wiederum der Nachteil darin, dass der Führungszapfen an der Innenseite der Führungshülse angeordnet ist, was in der Herstellung aufwändig und mit hohen Ausschussraten verbunden ist. Gleichfalls ist aber auch keine eindeutige Endlagenpositionierung der Kanüle mit deren Kanülenhalter in der aktivierten Stellung gegeben, bei welchem die Kanüle den Boden des Dichteinsatzes durchragt. Des Weiteren kann eine unbeabsichtigte Verlagerung der Kupplungsteils bezüglich des Kolbenstopfens erfolgen, wodurch die Dichtheit und vor allem die Sterilität nicht mehr gewährleistet sind.

Aus der US 6,613,016 B1 ist eine Injektionsspritze mit einem Spritzenzylinder zur Aufnahme eines abzugebenden Medikamentes bekannt geworden, bei welchem am distalen Ende des Spritzenzylinders eine Auspresseinheit und am proximalen Ende desselben ein Injektionsspritzenkopf angeordnet ist. Der Injektionsspritzenkopf weist ein im Spritzenzylinder gleitverschieblich gelagertes Halteelement für eine darin einsetzbare Nadelanordnung auf. Dabei steht die Nadelanordnung stets mit dem Innenraum des Spritzenzylinders in Strömungsverbindung. Die Auspresseinheit umfasst eine Kolbenstange mit einem fest damit verbundenen stangenförmigen Element, welches an der dem Innenraum des Spritzenzylinders zugewendeten Seite ein pfeilspitzenförmigbzw. kegelförmig ausgebildetes Ende aufweist. Auf diesem stangenförmig ausgebildeten Kolbenstangenkörper ist ein rohrförmiger Adapter gleitverschieblich gelagert, welcher in einer Ausgangsstellung über eine Arretier- bzw. Rastvorrichtung relativ bezüglich des stangenförmigen Kolbenstangenkörpers an diesem arretiert gehalten ist. An seinem dem Spritzenzylinder zugewendeten Ende ist der elastisch verformbare Kolbenstopfen gehalten bzw. angeordnet. Nach vollständigem Ausspritzen des abzugebenden Medikaments kommt die Arretiervorrichtung zwischen dem rohrförmigen Adapter und dem stangenförmigen Kolbenstangenkörper außer Eingriff und es durchsticht das pfeilförmige Ende des Kolbenstangenkörpers den Dichtstopfen und dringt in weiterer Folge in das Halteelement des Injektionsspritzkopfes ein. Dadurch erfolgt ein Kupplungsvorgang, wodurch der gesamte Injektionsspritzenkopf in den Innenraum des Spritzenzylinders zurückgezogen werden kann.

Die vorliegende Erfindung zielt darauf ab, eine Injektionsspritze mit einem Injektionsspritzenkopf sowie einer Auspresseinheit der einleitend angegebenen Art zu schaffen, welche diese Nachteile vermeidet, und insbesondere nach Verbinden der Kanüle mit dem Spritzenzylinder eine weitere Beweglichkeit der Kanüle verhindert. Ein weiteres Ziel der Erfindung liegt darin, die Kanüle mit dem Spritzenzylinder so zu verbinden, dass keine Abriebpartikel erzeugt werden können. Weiters soll auch noch eine verbesserte Kupplungsverbindung zwischen dem Kolbenstopfen und dem Kupplungsteil der Auspresseinheit geschaffen werden.

Der erfindungsgemäße Injektionsspritzenkopf erreicht dies dadurch, dass der Triebling zumindest eine Führungsnoppe aufweist, die wahlweise mit einer von zwei in der Führungshülse vorgesehenen Kulissenbahnen in Eingriff versetzbar ist, wobei die Kulissenbahnen gegensinnige Steigungen aufweisen.

Dabei ist vorteilhaft, dass die Ausbildung des Führungsnoppens formtechnisch einfacher erfolgen kann und dabei vor allem die Sicherheit der Funktion der gesamten Spritze erhöht werden kann. Weiters wird die Ausbildung der Kulissenbahnen ebenfalls einfacher und vor allem sicherer in der Anwendung. Damit wird eine kompakte und vor allem sicher und einfach zu bedienende Injektionsspritze geschaffen, welche auch durch medizinisch nicht vorgebildete Personen angewendet werden kann und dabei eine hohe Betriebssicherheit aufweist.

Eine weitere Ausbildung sieht vor, dass der Triebling zwei Führungsnoppen umfasst, welche in einer senkrecht zu einer Längsachse ausgerichteten Ebene sowie in Umfangsrichtung voneinander distanziert angeordnet sind und zur Verlagerung der Nadeleinheit von der Ruhestellung hin zur Aktivierungsstellung nur einer der beiden Führungsnoppen mit einer der beiden Kulissenbahnen in Eingriff versetzbar ist. Dadurch wird die Betriebssicherheit zusätzlich erhöht und eine noch bessere Betätigungsmöglichkeit geschaffen.

Eine weitere bevorzugte Ausführungsform hat die Merkmale, dass in der Führungshülse in einem Umfangsbereich zwischen den beiden Kulissenbahnen eine Freistellung ausgebildet ist und die Freistellung zur Aufnahme jenes Führungsnoppens dient, welcher während der Verstellung der Nadeleinheit mit keiner der Kulissenbahnen in Eingriff steht. Damit kann trotz der beiden Führungsnoppen einerseits eine exakte Führung eines der beiden Noppen geschaffen werden und andererseits ein Verklemmen während der Drehbewegung verhindert werden. PA3

Eine andere Ausführungsform zeichnet sich dadurch aus, dass die Nadeleinheit, insbesondere deren Kanülenhalter, und die Führungshülse in der Aktivierungsstellung mittels zusammenwirkender Arretierelemente einer Arretiervorrichtung in Axialrichtung relativ zueinander positioniert gehalten sind. Damit wird vor, bei und nach der Abgabe des Medikaments stets eine gesicherte Lage der Kanüle erzielt, und eine ungewollte Axialverlagerung vermieden.

Eine weitere Ausbildung sieht vor, dass die Nadeleinheit, insbesondere deren Kanülenhalter, mit dem Triebling um die Längsachse drehbar, jedoch in Axialrichtung arretiert gekuppelt ist. Damit kann eine Vormontage des der Kanüle samt Kanülenhalter im Triebling erfolgen, ohne dass die notwendige Bewegungsfreiheit der Drehbarkeit eingeschränkt wird.

Eine weitere bevorzugte Ausführungsform hat die Merkmale, dass die Nadeleinheit, insbesondere deren Kanülenhalter, in der Führungshülse in Axialrichtung geradlinig geführt gelagert ist. Damit wird eine reine Durchstichbewegung im Bereich des Dichtelements erzielt und das Herauslösen von Partikel des Dichtelements vermieden. Damit wird eine noch sicherere Abgabe des Medikament im Hinblick auf Reinheit erzielt.

Eine andere Ausführungsform zeichnet sich dadurch aus, dass am Ende jeder Kulissenbahn eine Kulissenerweiterung vorgesehen ist, in welche der Führungsnoppen einrastbar ist. Bei Vorhandensein eines zusätzlichen Rasthakens kann auch noch dieser in der Kulissenerweiterung einrastbar ausgebildet sein.

Eine weitere bevorzugte Ausführungsform ist dadurch gekennzeichnet, dass das distale Ende der Führungshülse als Kupplung ausgebildet ist. Alternativ dazu kann das distale Ende der Führungshülse flach ausgebildet sein.

Eine weitere Ausbildung sieht vor, dass an einer Außenseite der Führungshülse zumindest eine Längsrippe angeordnet ist, welche verdrehfest an einem Rastorgan einer den Injektionsspritzenkopf aufnehmenden Schutzkappe abgestützt ist. Damit wird eine Verdrehung der Führungshülse während dem Aktivierungsvorgang verhindert.

Eine weitere bevorzugte Ausführungsform ist dadurch gekennzeichnet, dass die Auspresseinheit eine Kolbenstange, einen Kupplungsteil mit einem Basiskörper und einem auf die von der Kolbenstange abgewendete Seite vorragenden Kupplungsfortsatz sowie einen Kolbenstopfen umfasst, wobei der Basiskörper des Kupplungsteils mit der Kolbenstange lösbar gekuppelt ist, und der Kolbenstopfen ein sich ausgehend von einem der Kolbenstange zugewendeten Ende hinein erstreckendes Sackloch aufweist, das mit einem Boden verschlossen ist, wobei der Kupplungsfortsatz des Kupplungsteils in das Sackloch hineinragt und in einer unbetätigten Ausgansstellung der Kolbenstopfen in axialer Richtung vom Basiskörper distanziert angeordnet ist und weiters der Boden des Sackloches von einem Kupplungsfortsatzende des Kupplungsfortsatzes undurchstochen ist, wobei am Kupplungsfortsatz zumindest ein erstes Rastelement angeordnet ist, welches in der unbetätigten Ausgansstellung in eine im Kolbenstopfen im Bereich des Sackloches angeordnete Rastausnehmung eingesetzt ist. Die sich daraus ergebenden Vorteile liegen darin, dass so eine Vormontage und vor allem ein sicheres Einsetzen des Kolbenstopfens in den Spritzenzylinder erfolgen können. Diese in Axialrichtung wirkende Halterung kann zum Auspressen des Medikaments überwunden werden und dient auch während des Transports und der Lagerung dazu, dass der Kolbenstopfen im Bereich seines Sackloches unversehrt erhalten bleibt. Damit ist auch bei Erschütterungen eine ungewollte Verlagerung vermieden.

Eine andere Ausführungsform zeichnet sich dadurch aus, dass das erste Rastelement bei aneinander liegender Stellung des Basiskörpers und des Kolbenstopfens am Boden des Sackloches in dem vom Kupplungsfortsatz durchsetzten Bereich anliegt. Dadurch wird eine Abstützwirkung des durchstochenen Bodens erzielt und so die Dichtheit auch beim Auspressen des Medikaments in diesem Abschnitt sicher gestellt.

Eine weitere Ausbildung sieht vor, dass am Kupplungsfortsatz zwischen dem Basiskörper und dem ersten Rastelement ein weiteres Rastelement angeordnet ist, welches bei der Rückstellbewegung der Auspresseinheit an einer im Sackloch des Kolbenstopfens ausgebildeten Schulter anliegend abgestützt ist. Damit wird die Rückstellbewegung erleichtert und ein Absteifen des weiters mitgezogenen Injektionsspritzenkopfes verhindert.

Eine andere Ausführungsform zeichnet sich dadurch aus, dass das Kupplungsfortsatzende auf die von der Kolbenstange abgewendete Seite pfeilförmig verjüngend ausgebildet ist und zumindest einen, bevorzugt zwei, den Querschnitt des Kupplungsfortsatzes überragende Rastarme umfasst. Damit wird für die Kupplungsbewegung die Eindringkraft herabgesetzt und darüber hinaus auch noch die Rückhaltekraft durch die Ausbildung einer stabileren Schulter im Dichtelement erhöht. Weiters kann dadurch gerade bei mit einem Gleitmittel beschichtetem Dichtelement eine bessere Verankerungswirkung bzw. ein Verkrallen des oder der Rastarme durch das bereichsweise Eindringen in den elastischen Werkstoff des Dichtelements erzielt werden.

Eine weitere Ausbildung sieht vor, dass der Basiskörper des Kupplungsteils auf der der Kolbenstange zugewendeten Seite elastisch verformbare Kupplungsarme umfasst, die mit einem an der Kolbenstange ausgebildeten Kupplungselement lösbar gekuppelt sind. Damit kann die Entkupplung der Kolbenstange nach der Rückstellbewegung der gesamten Nadeleinheit einfach erfolgen.

Eine weitere bevorzugte Ausführungsform ist dadurch gekennzeichnet, dass die Auspresseinheit weiters noch ein Handhabungselement umfasst, welches mit einem distalen Ende eines Spritzenzylinders kuppelbar ist, und eine lichte innere Weite eines Durchtrittskanals im Handhabungselement in einem distalen Abschnitt größer ausgebildet ist als eine äußere Querschnittsabmessung der Kupplungsarme in deren unverformten Stellung. Damit kann der Spritzenzylinder einfach mit einer nahezu gleichen Innenabmessung über den größten Teil seiner Baulänge ausgebildet werden. Dadurch werden Formkosten und Ungenauigkeiten in der Fertigung des Spritzenzylinders vermieden. Durch das zusätzlich ankuppelbare Handhabungselement kann die Betriebssicherheit erhöht und vor allem die Wiederverwendung durch einfacher auszubildende Bauelemente verhindert werden.

Eine andere Ausführungsform zeichnet sich dadurch aus, dass zwischen dem Basiskörper des Kupplungsteils und dem Handhabungselement eine Haltevorrichtung mit zusammenwirkenden Halteelementen ausgebildet ist, welche bei sich in Eingriff befindlichen Halteelementen zumindest eine axiale Verstellung des Kupplungsteils in proximaler Richtung verhindern. Damit kann vor allem die unbeabsichtigte Wiederverwendung und damit verbunden die mögliche Übertragung von Krankheiten verhindert werden.

Eine weitere Ausbildung sieht vor, dass am Handhabungselement im Bereich seines distalen Endes zumindest ein Leitelement angeordnet ist, welches in den Querschnitt des Durchtrittskanals hineinragt. So wird auch nach dem Abkuppeln der Kolbenstange vom Kupplungsteil mit der geschützten Nadeleinheit ein erneutes Einsetzen und eine Wiederverwendung verhindert.

Eine andere Ausführungsform zeichnet sich dadurch aus, dass zwischen dem Handhabungselement und der Kolbenstange eine Rückstellvorrichtung vorgesehen ist, mit welcher die Kolbenstange, der Kupplungsteil, der Kolbenstopfen sowie die damit bedarfsweise kuppelbare Nadeleinheit in distaler Richtung relativ bezüglich des Spritzenzylinders rückverstellbar sind. Dadurch kann nach der Freigabe bzw. Auslösung der Rückstellvorrichtung eine automatisierte Schutzstellung der gebrauchten Kanüle innerhalb des Spritzenzylinders erreicht werden.

Eine weitere Ausbildung sieht vor, dass die Rückstellvorrichtung zumindest ein Federelement umfasst, welches mit seinem proximalen Ende am Handhebungselement und mit seinem distalen Ende an einem rohrförmigen Gleitkörper abgestützt ist und der Gleitkörper in der vorgespannten Stellung des Federelements am Handhabungselement mittels einer Rückhaltevorrichtung bedarfsweise lösbar gehalten ist. So kann die aufgebaute bzw. gespeicherte Rückstellenergie während des Abgabevorganges unverändert aufrecht erhalten werden und es kann die Bewegung der Kolbenstange für den Abgabevorgang ungehindert erfolgen.

Eine weitere bevorzugte Ausführungsform hat die Merkmale, dass die Freigabe der Rückhaltevorrichtung nach einem vorbestimmten Verstellweg der Kolbenstange relativ bezüglich des Spritzenzylinders durch zusammenwirkende Stellelemente erfolgt, wobei zumindest ein erstes Stellelement am Gleitkörper und zumindest ein weiteres Stellelement an der Kolbenstange angeordnet ist. Damit kann ohne zusätzliches Zutun des Benutzers der Injektionsspritze die automatisierte Auslösung der Rückstellvorrichtung erfolgen. Damit kann ein sogenannter passiver Nadelrückzug, insbesondere der Kanüle, in den Spritzenzylinder erzielt werden. Damit kann in Abhängigkeit vom vorgegebenen bzw. vorbestimmten Verstellweg, welcher unbedingt notwendig ist, um die Stellelemente miteinander in Wirkeingriff zu bringen, einerseits die minimale Abgabemenge des Medikaments festgelegt und andererseits der Benutzer von einer unbedingt durchzuführenden Auslösebewegung für die Rückstellung der Kanüle in deren Schutzstellung befreit werden.

Eine andere Ausführungsform zeichnet sich dadurch aus, dass der Werkstoff des Spritzenzylinders aus Glas gewählt ist und im Spritzeninnenraum ein Medikament eingefüllt und für die Abgabe bereitgestellt ist, wobei der das Medikament aufnehmende Spritzeninnenraum an seinem proximalen Ende vollständig durch das bis hin zur Aktivierung undurchstochene Dichtelement und an seinem distalen Ende durch den ebenfalls noch undurchstochenen Kolbenstopfen gegen die Innenwand des Spritzenzylinder bakteriendicht abgedichtet ist. Somit handelt es sich dabei um einen Spritzentyp, bei welchem bereits das Medikament eingefüllt und für die Abgabe bereitgestellt ist. Dabei spricht man von einer Fertigspritze, insbesondere Glasfertigspritze. So durchsticht erst nach der Aktivierung des Injektionsspritzenkopfes durch Verdrehen der Schutzkappenspitze das distale Ende der Kanüle den Boden des Dichtelements und stellt somit die Leitungsverbindung mit dem Innenraum des Spritzenzylinders und damit verbunden dem Medikament her. Dann kann die Abgabe des bevorrateten Medikaments erfolgen.

Die Erfindung wird nachstehend anhand von in den Zeichnungen dargestellten Ausführungsbeispielen näher erläutert.

Es zeigen jeweils in stark schematisch vereinfachter Darstellung:
- Fig. 1: eine gesprengte Darstellung des Injektionsspritzenkopfes;
- Fig. 2: eine gesprengte Darstellung des Injektionsspritzenkopfes um 180° axial gedreht;
- Fig. 3: einen Injektionsspritzenkopf um 90° axial gedreht;
- Fig. 4: eine gesprengte Darstellung einer Spritze in einer alternativen Ausführungsform;
- Fig. 5: einen Schnitt durch den Spritzenkopf einer alternativen Ausführungsform;
- Fig. 6: eine weitere mögliche Ausbildung einer Injektionsspritze in ungeöffneter Originalstellung, in Ansicht geschnitten;
- Fig. 7: den Injektionsspritzenkopf der Injektionsspritze nach der Aktivierung der Injektionsspritze nach Fig. 6, in Ansicht geschnitten;
- Fig. 8: die Auspresseinheit der Injektionsspritze nach der Aktivierung der Injektionsspritze nach Fig. 6, in Ansicht geschnitten;
- Fig. 9: die Entsorgungsstellung der Injektionsspritze nach den Fig. 6 bis 8, in Ansicht geschnitten;
- Fig. 10: die Auspresseinheit und den Spritzenzylinder mit Handhabungselement nach den Fig. 6 bis 9 in Explosionsdarstellung, in Ansicht geschnitten;
- Fig. 11: den Injektionsspritzenkopf und die Schutzkappe nach den Fig. 6 bis 9 in Explosionsdarstellung, in Ansicht geschnitten;
- Fig. 12: die Führungshülse des Injektionsspritzenkopfes nach den Fig. 6 bis 11, in schaubildlicher Darstellung;
- Fig. 13: eine weitere und gegebenenfalls für sich eigenständige Ausbildung einer Injektionsspritze mit einer automatisierten passiven Rückstellvorrichtung für die Nadeleinheit in der Ausgangsstellung, in Ansicht geschnitten;
- Fig. 14: die Injektionsspritze nach Fig. 13, während des Abgabevorganges des Medikaments, in Ansicht geschnitten;
- Fig. 15: die Injektionsspritze nach den Fig. 13 und 14, beim Auslösevorgang der Rückstellvorrichtung, in Ansicht geschnitten;
- Fig. 16: die Injektionsspritze nach den Fig. 13 bis 15, in der Schutzstellung der Nadeleinheit innerhalb des Spritzenzylinders, in Ansicht geschnitten.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen.

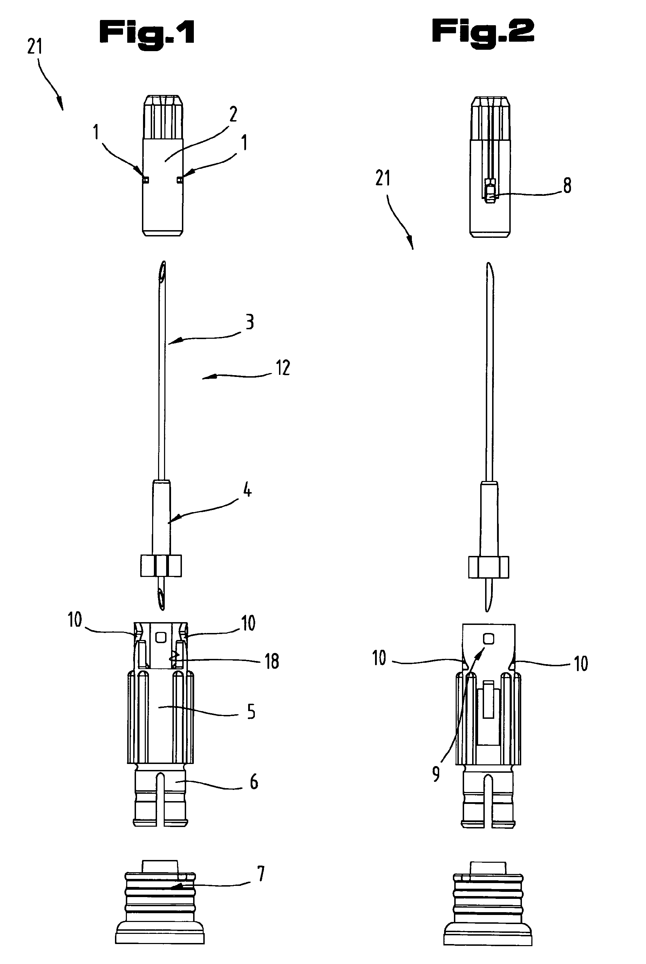

Ein Injektionsspritzenkopf 21 weist gemäß Fig. 1 eine Nadeleinheit 12 mit einer Kanüle 3 und einem Kanülenhalter 4 auf. Die Nadeleinheit 12 wird in eine Führungshülse 5 eingesetzt. Der Antrieb der Nadeleinheit 12 in Richtung eines nicht gezeigten Spritzenzylinders, um die Kanüle 3 unmittelbar vor der Injektion mit dem versiegelten Spritzenzylinder zu verbinden, erfolgt durch einen Triebling 2, welcher auf die Nadeleinheit 12 gesteckt wird. Der Triebling 2, der Kanülenhalter 4 und die Führungshülse 5 sind vorzugsweise aus Kunststoff hergestellt.

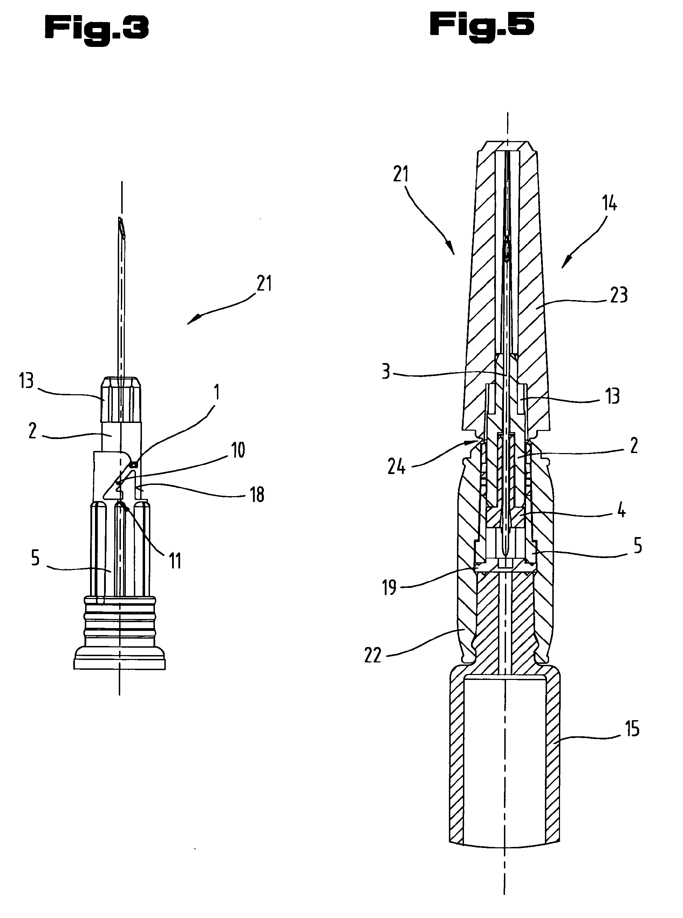

In Fig. 3 ist der fertig zusammengesteckte Injektionsspritzenkopf 21 dargestellt. Der Triebling 2 weist zwei Führungsnoppen 1 auf, die im unbetätigten Zustand des Injektionsspritzenkopfes 21 vor dem Eingang jeweils einer Kulissenbahn 10 angeordnet sind. Die Kulissenbahnen 10 sind Ausnehmungen in der Führungshülse 5. Sie verlaufen auf der Führungshülse 5 mit jeweils gegensinniger Steigung. Wird der Triebling 2 in eine Drehbewegung gebracht, was beispielsweise dadurch erfolgen kann, dass Lamellen einer in Fig. 1 bis 3 nicht dargestellten Schutzkappe in längs einer Längsachse 19 verlaufende Stege 13 des Trieblings 2 eingreifen, wird einer der Führungsnoppen 1 in eine der Kulissenbahnen 10 geführt, während der andere Führungsnoppen 1 ungehindert in einen Freiraum bzw. eine Freistellung 18 der Führungshülse 5 eintritt. Der Führungsnoppen 1, der in eine Kulissenbahn 10 geführt ist, folgt der Steigung dieser Kulissenbahn 10 und bewegt den Triebling 2 zusammen mit der Nadeleinheit 12 in Richtung des Spritzenzylinders. Auf diese Weise wird die Nadeleinheit 12 sowohl bei einer Linksdrehung als auch bei einer Rechtsdrehung zum Spritzenzylinder hin bewegt. Der Umfang der Nadeleinheit 12 und der entsprechend geformte innere Kanal der Führungshülse 5 sind so ausgebildet, dass die Nadeleinheit 12 bei Antrieb durch den Triebling 2 keine Drehung ausführen kann, sondern linear in Richtung zum Spritzenzylinder hin bewegt wird. Gemäß den Fig. 1 bis 3 erfolgt die Drehung des Trieblings 2 zur vollständigen Bewegung der Nadeleinheit 12 um ca. 90°.

Der Triebling 2 weist weiters gemäß Fig. 2 einen Rasthaken 8 auf, der etwa auf der Höhe der Führungsnoppen 1 auf der anderen Umfangsseite des Trieblings 2 angeordnet ist. Im unbetätigten Zustand des Injektionsspritzenkopfes 21 ist der Rasthaken 8 in einem Fenster 9 der Führungshülse 5 positioniert. Den Rasthaken 8 aus dieser Position herauszuführen, erfordert die Überwindung eines mechanischen Widerstandes, wodurch der Injektionsspritzenkopf 21 in seinem unbetätigten Zustand gegen unbeabsichtigtes Betätigen gesichert ist. Bei Drehung des Trieblings 2 wandert der Rasthaken 8 bis zum Ende derjenigen Kulissenbahn 10, die nicht durch einen Führungsnoppen 1 belegt ist. Die Kulissenbahnen 10 weisen jeweils gemäß Fig. 3 eine horizontale Kulissenerweiterung 11 auf. Nach Vollendung der Trieblingsdrehung befinden sich in einer Kulissenerweiterung 11 ein Führungsnoppen 1 und in der anderen Kulissenerweiterung 11 der Rasthaken 8. Ein Herausdrehen des Trieblings 2 und somit eine weitere Bewegung der Nadeleinheit 12 ist jetzt nicht mehr möglich, da die gegensinnigen Steigungen der Kulissenbahnen 10 die Bewegungen des Führungsnoppens 1 und des Rasthakens 8 gegenseitig blockieren. Dies begünstigt den sicheren Umgang mit der Injektionsspritze, da ein mehrmaliges Verbinden und Lösen der Kanüle von dem Spritzenzylinder unmöglich ist.

Der Injektionsspritzenkopf 21 weist in einer ersten Ausführungsform gemäß der Fig. 1 bis 3 an seinem distalen Ende eine Kupplung 6 auf, die vorzugsweise als distales Ende der Führungshülse 5 ausgebildet ist. Die Kupplung 6 kann zum Beispiel mit einem Dichtelement 7 des Spritzenzylinders in Form einer Steckverbindung zusammengefügt werden. Das distale Ende des Dichtelements 7 kann fest mit dem Spritzenzylinder verbunden oder Teil einer in den Spritzenzylinder nach der Injektion einziehbaren Baugruppe sein. Dadurch ist der Injektionsspritzenkopf 21 auf mehrere Injektionsspritzentypen aufsteckbar.

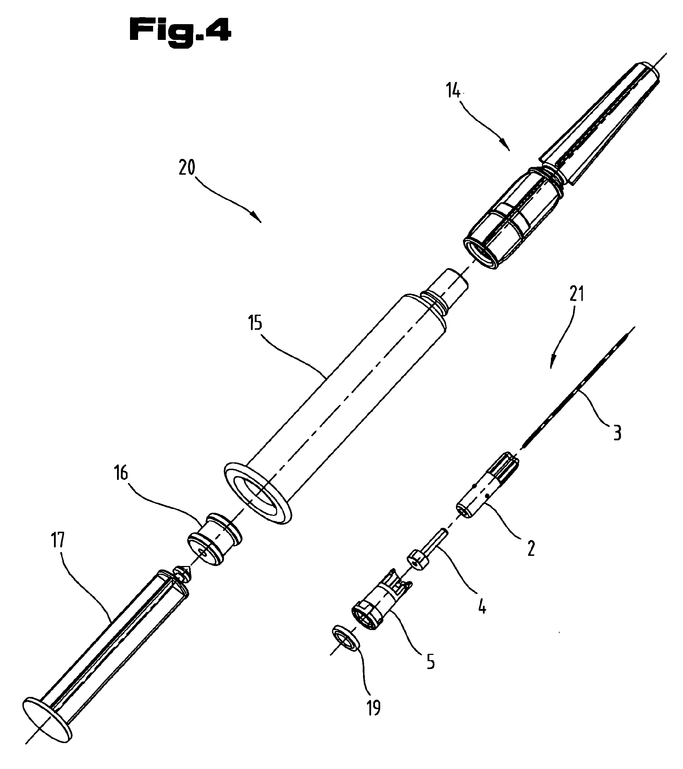

Fig. 4 zeigt eine vollständige Injektionsspritze 20 gemäß der Erfindung in einer zweiten Ausführungsform. Das distale Ende der Führungshülse 5 ist flach ausgebildet und schließt an eine Dichtscheibe 19 an. Im zusammengesetzten Zustand liegt die Dichtscheibe 19 auf dem proximalen Ende eines Spritzenzylinders 15 auf, in welchen eine Kolbenstange 17 und ein Kolbenstopfen 16 zum Bewegen der Flüssigkeit eingesteckt sind. Der Spritzenkopf des Spritzenzylinders 15 mitsamt dem Injektionsspritzenkopf 21 werden durch eine Schutzkappe 14 abgedeckt.

Gemäß Fig. 5 wird die Schutzkappe 14 auf den Spritzenzylinder 15 aufgesteckt und presst dadurch die Führungshülse 5 auf die Dichtscheibe 19 und das proximale Ende des Spritzenzylinders 15. Die Schutzkappe 14 umfasst in bekannter Weise eine Schutzkappenbasis 22 sowie eine Schutzkappenspitze 23, welche über eine Sollbruchstelle 24 verbunden sind. Durch Drehen der Schutzkappenspitze 23 wird die Sollbruchstelle 24 zwischen dieser und der Schutzkappenbasis 23 gebrochen. Innere Lamellen der Schutzkappenspitze 23 greifen in die Stege 13 des Trieblings 2 und versetzen damit auch ihn in eine Drehbewegung. Somit wird schließlich der Triebling 2 und die Nadeleinheit 12 in der oben beschriebenen Weise in Richtung des Spritzenzylinders 15 bewegt und die Dichtscheibe 19 durchstochen.

Unabhängig davon wäre es auch noch möglich, mit nur einer einzigen Führungsnoppe 1 am Triebling 2 das Auslangen zu finden und diese Führungsnoppe 1 wahlweise in eine der beiden gegensinnig ausgebildeten Kulissenbahnen 10 je nach gewählter Drehrichtung (im Uhrzeigersinn oder gegen dem Uhrzeigersinn) in Eingriff zu bringen und so die damit verbundenen Verstellung des Kanülenhalters 4 mitsamt der Kanüle 3 hin in Richtung auf den Spritzenzylinder 15 zu bewirken. Weiters könnte auch noch auf die Anordnung bzw. das Vorsehen des Rasthakens 8 in Verbindung mit dem Fenster 9 überhaupt verzichtet werden, wodurch einfachere Formen erzielbar sind.

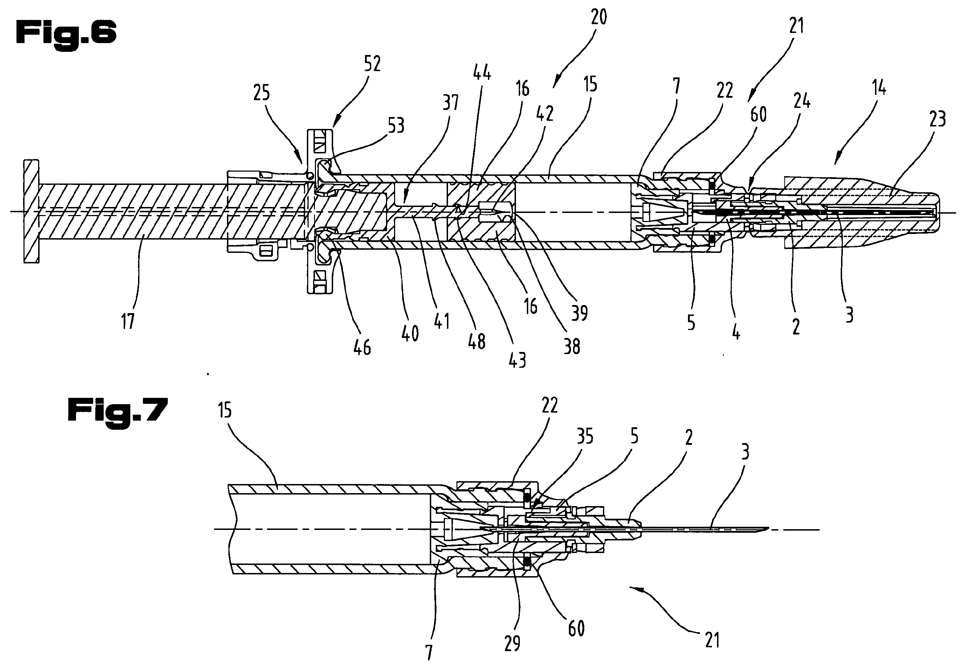

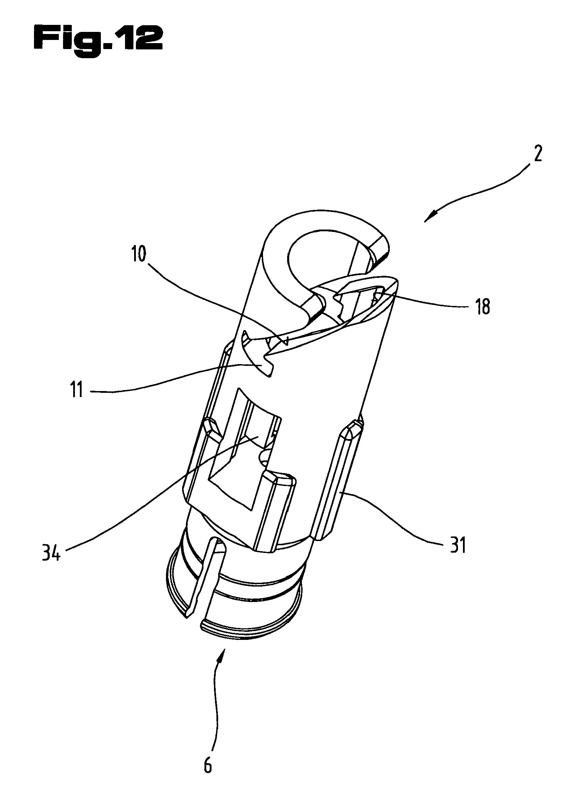

In den Fig. 6 bis 12 ist eine weitere und gegebenenfalls für sich eigenständige Ausführungsform einer Injektionsspritze 20 gezeigt, wobei wiederum für gleiche Teile gleiche Bezugszeichen bzw. Bauteilbezeichnungen wie in den vorangegangenen Fig. 1 bis 5 verwendet werden. Um unnötige Wiederholungen zu vermeiden, wird auf die detaillierte Beschreibung in den vorangegangenen Fig. 1 bis 5 hingewiesen bzw. Bezug genommen.

Diese hier gezeigte weitere Ausführungsform der Injektionsspritze 20, insbesondere deren Injektionsspritzenkopf 21, ist ähnlich ausgebildet wie jener Injektionsspritzenkopf 21, welcher in den Fig. 1 bis 3 beschrieben worden ist.

So umfasst die Injektionsspritze 20 den Spritzenzylinder 15 zur Aufnahme des abzugebenden Medikaments, bei welchem an seinem proximalen Ende der Injektionsspritzenkopf 21 und in seinem distalen Ende eine Auspresseinheit 25 angeordnet ist. Der Injektionsspritzenkopf 21 ist wiederum durch die Schutzkappe 14 abgedeckt und schützt somit die in der Ruhestellung bzw. Ausgangsstellung vorragende proximale Spitze der Kanüle 3 vor Verunreinigungen sowie ungewollten Stichverletzungen. Die Schutzkappe 14 mit ihrer Schutzkappenbasis 22 ist auf das proximale Ende des Spritzenzylinders 15 aufgesteckt bzw. aufgeschnappt und bevorzugt durch die radiale Vorspannung der Schutzkappenbasis 22 nahezu verdrehfest daran gehaltert. Durch entsprechende miteinander in Eingriff stehende Haltemittel ist weiters die Schutzkappenbasis 22 in axialer Richtung am proximalen Ende des Spritzenzylinders 15 gehalten. Weiters ist noch bevorzugt zwischen dem proximalen Ende des Spritzenzylinders 15, insbesondere dessen Stirnfläche, und einer radial in Richtung auf die Längsachse vorspringenden Schulter der Schutzkappenbasis 22 ein Dichtring 60 angeordnet. Damit wird auch im Übergangsbereich des Spritzenzylinders 15 und der Schutzkappe 14 ein bakteriendichter Abschluss erzielt.

Am Triebling 2 sind bei diesem hier gezeigten Ausführungsbeispiel zwei Führungsnoppen 1 angeordnet, welche in einer senkrecht zur Längsachse ausgerichteten Ebene sowie in Umfangsrichtung voneinander distanziert angeordnet sind. Diese sind zur Verlagerung der Nadeleinheit 12 von der Ruhestellung hin zur Aktivierungsstellung mit nur einer der beiden Kulissenbahnen 10 in Eingriff versetzbar, wodurch die Aktivierung und die damit verbundene axiale Längsverstellung der Kanüle 3 mitsamt dem Kanülenhalter 4 hin in Richtung zum Spritzenzylinder 15 erfolgt und dabei das Dichtelement 7 im Bereich seines proximalen Endes von der Kanülenspitze durchstochen wird. Damit wird eine Leitungsverbindung zwischen dem Innenraum des Spritzenzylinders 15 und der Kanüle 3 geschaffen. Der Antrieb des Trieblings 2 erfolgt durch entsprechende Drehbewegung der Schutzkappenspitze 23 um die Längsachse. Dabei stehen der oder die Stege 13 des Trieblings 2 mit den zuvor beschriebenen inneren Lamellen der Schutzkappenspitze 23 in Eingriff.

Wie nun besser aus einer Zusammenschau der Fig. 6, 7 sowie 11 zu ersehen ist, ist im Bereich des proximalen Endes der Führungshülse 5 jener Bereich vorgesehen, welcher die beiden Kulissenbahnen 10 ausbildet. Diese beiden zueinander gegensinnig ausgerichtet verlaufenden Kulissenbahnen 10 sind in Umfangsrichtung versetzt zueinander angeordnet, um mit einem der beiden Führungsnoppen 1 bei einer entsprechenden Aktivierung in Eingriff gebracht zu werden. Um ausreichend Bewegungsfreiheit für den weiteren Führungsnoppen 1 zu schaffen, welcher sich außer Eingriff mit einer der beiden Kulissenbahnen 10 befindet, ist hier in einem Umfangsbereich zwischen den beiden Kulissenbahnen 10 die Freistellung 18 ausgebildet und dient zur Aufnahme einer der beiden Führungsnoppen 1. Im vorliegenden Ausführungsbeispiel ist die Freistellung 18 durch eine radiale Vergrößerung und einer damit verbundenen Abnahme der Wandstärke der Führungshülse 5 ausgebildet. Die beiden Kulissenbahnen 10 werden dadurch als bevorzugt schlitzförmige Ausnehmungen bzw. Durchbrüche in der Außenwand der Führungshülse 5 gebildet und bieten so eine noch bessere Führung eines der beiden Führungsnoppen 1 aus. Am Ende der Kulissenbahnen 10 ist wiederum die zuvor beschriebene bevorzugt waagrecht ausgebildete Kulissenerweiterung 11 ausgebildet, in welcher eine der beiden Führungsnoppen 1 nach entsprechender Verlagerung in der Aktivierungsstellung angeordnet ist.

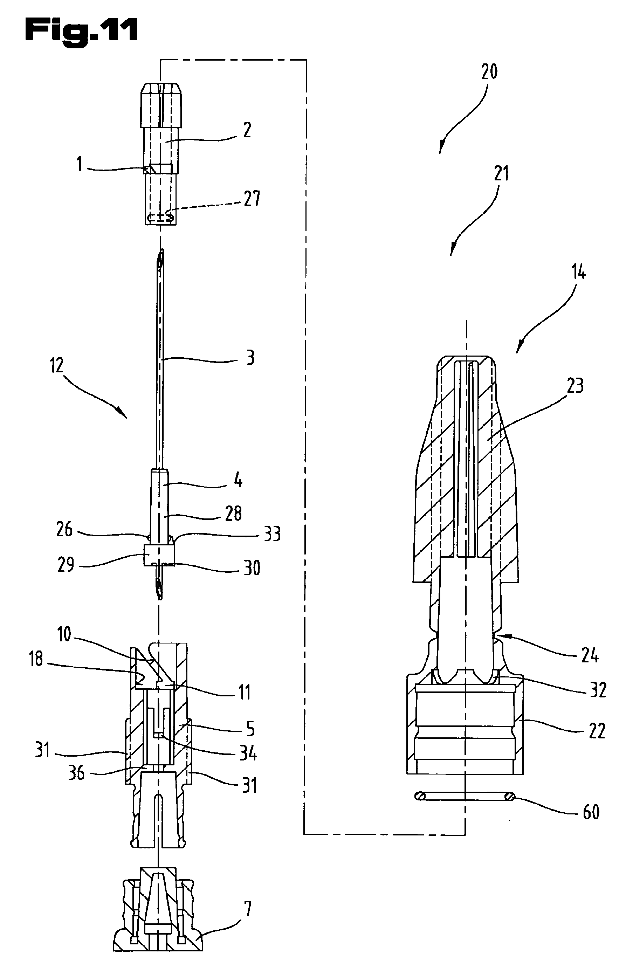

Um eine gewisse Erleichterung für den Montagevorgang zu erzielen, ist es vorteilhaft, wenn die Nadeleinheit 12, insbesondere der Kanülenhalter 4, mit dem Triebling 2 um die Längsachse drehbar, jedoch in Axialrichtung arretiert gekuppelt ist. Dazu ist, wie dies am besten aus der Fig. 11 zu ersehen ist, zumindest ein radial vorragender Vorsprung 26 am Schaft 28 des Kanülenhalters 4 angeordnet, welcher mit einer bevorzugt radial umlaufenden Ausnehmung 27 im Triebling 2 in Eingriff gebracht werden kann. Bevorzugt kann der Vorsprung 26 auch umlaufend ausgebildet sein, welcher in eine bevorzugt ringförmige Nut, welche die Ausnehmung 27 ausbildet, zur gegenseitigen Arretierung eingreift.

Es wäre aber unabhängig davon auch möglich, die Ausnehmung 27 im Schaft 28 des Kanülenhalters 4 und den oder die Vorsprünge 26 am Triebling 2 vorzusehen.

Am Schaft 28 des Kanülenhalters 4 ist im Bereich seines distalen Endes ein Ansatz 29 ausgebildet, welcher den Schaft 28 radial überragt. Dieser bevorzugt ringförmig ausgebildete Ansatz 29 kann mit seinem proximalen Ende am distalen Ende des Trieblings 2 in der arretierten bzw. gehaltenen Stellung zur Anlage gebracht werden.

Um eine exakte geradlinige Axialbewegung der Kanüle 3 während deren Axialverstellung in der Führungshülse 5 zu erzielen, ist die Nadeleinheit 12, insbesondere der Kanülenhalter 4, in der Führungshülse 5 entsprechend geradlinig zu führen. Dazu kann beispielsweise am Ansatz 29 des Kanülenhalters 4 zumindest ein Führungsorgan 30 angeordnet sein, welches mit einer nicht näher dargestellten Führungsbahn an der Innenseite der Führungshülse 5 in Führungseingriff steht. Bevorzugt können auch mehrere Führungsorgane 30 mit damit zusammenwirkenden Führungsbahnen in der Führungshülse 5 vorgesehen sein. Weiters kann auch noch der Kanülenhalter 4 nach dem Einsetzen in die Führungshülse 5 in der Ruhe- bzw. Ausgangsstellung in Richtung auf die proximale Seite hin in Axialrichtung arretiert gekuppelt sein. Damit wird auch beim Montagevorgang zusätzlich zur Längsführung eine gewisse Kupplungswirkung erzielt und ein unbeabsichtigtes Lösen von der Führungshülse 5 verhindert.

Wie bereits zuvor beschrieben, ist die Schutzkappenbasis 22 der Schutzkappe 14 auf das proximale Ende des Spritzenzylinders 15 aufgeschnappt und durch die radiale Vorspannung derselben nahezu verdrehfest daran gehalten. Um auch eine relative Verlagerung der Führungshülse 5 bezüglich der Schutzkappe 14, insbesondere deren Schutzkappenbasis 22, zu verhindern, ist an einer Außenseite der Führungshülse 5 zumindest eine Längsrippe 31 angeordnet bzw. ausgebildet. Ein Abschnitt der Führungshülse 5 ist in einer Durchgangsöffnung der Schutzkappenbasis 22 aufgenommen, wobei sich proximale Enden der Längsrippen 31 an dafür vorgesehenen Rastorganen 32 an der Innenseite der den Injektionsspritzenkopf 21 aufnehmenden Schutzkappe 14 abstützen. Durch das Zusammenwirken der bevorzugt mehreren Längsrippen 31 mit dem oder den Rastorganen 32 ist die Führungshülse 5 relativ bezüglich der Schutzkappe 14 in ihrer Drehung um die Längsachse arretiert gehalten.

Damit kann nun, wie bereits zu vor beschrieben, die Aktivierung der Nadeleinheit 12 erfolgen, indem dass die Schutzkappenspitze 23 um die Längsachse in eine der beiden Richtungen - im Uhrzeigersinn oder gegen den Uhrzeigersinn -verdreht wird und dadurch die Sollbruchstelle 24 zwischen der Schutzkappenspitze 23 und der Schutzkappenbasis 22 durchtrennt wird. Durch die Antriebsverbindung der Schutzkappenspitze 23 mit dem Triebling 2 und in weiterer Folge der geführten Verstellung des Trieblings 2 mit seiner Führungsnoppe 1 in einer der Kulissenbahnen 10 kommt es zu der axialen Verlagerung der Kanüle 3 mitsamt dem Kanülenhalter 4 in distaler Richtung hin zum Spritzenzylinder 15. Dabei gleitet der Kanülenhalter 4 in Axialrichtung geradlinig geführt in der Führungshülse 5 hin zum Dichtelement 7 und durchsticht dieses.

Ist diese Aktivierungsstellung erreicht, erfolgt eine axiale Halterung bzw. Arretierung der Nadeleinheit 12, insbesondere des Kanülenhalters 4, an der Führungshülse 5. Diese erfolgt dadurch, dass einerseits der Ansatz 29 des Kanülenhalters 4 an seiner proximalen Seite ein erstes Arretierelement 33 ausbildet. Ein weiteres Arretierelement 34 ist an der Führungshülse 5 ausgebildet bzw. angeordnet und bevorzugt an einem radial elastisch verformbaren Federarm angeordnet. Das weitere Arretierelement 34 hintergreift das erste Arretierelement 33 in der Aktivierungsstellung der Nadeleinheit 12, wodurch eine axiale Halterung der Nadeleinheit 12 in proximaler Richtung erzielt wird. Die beiden Arretierelemente 33, 34 bilden in der Aktivierungsstellung eine Arretiervorrichtung 35 aus. Die Nadeleinheit 12, insbesondere der Ansatz 29, kommt in der Aktivierungsstellung an einem im Inneren der Führungshülse 5 angeordneten Bodenelement 36 zur Anlage. Im Bodenelement 36 ist eine Durchtrittsöffnung für den Durchtritt des distalen Endes der Kanüle 3 vorgesehen. So ist in der Aktivierungsstellung die Nadeleinheit 12 mit ihrem Ansatz 29 in axialer Richtung beidseits arretiert gehalten. Einerseits durch das Bodenelement 36 und andererseits durch das Zusammenwirken der Arretierelement 33, 34. Durch das Arretierelement 34 kann eine proximale Axialverlagerung des Kanülenhalters 4 verhindert werden, wodurch die Abgabe des Medikaments mit der Auspresseinheit 25 erfolgen kann, ohne dass eine ungewollte Ablösung bzw. Distanzierung der Kanüle 3 von der Injektionsspritze 20 erfolgt.

Aus einer Zusammenschau der Fig. 6, 8 und 10 ist die Auspresseinheit 25 im Bereich des distalen Endes des Spritzenzylinders 15 zu ersehen, welche ihrerseits einen Kolbenstopfen 16, eine Kolbenstange 17 sowie ein die Kolbenstange 17 mit dem Kolbenstopfen 16 kuppelndes bzw. verbindendes Kupplungsteil 37 umfasst. Der Kolbenstopfen 16 weist ausgehend von einem der Kolbenstange 17 zugewendeten Ende ein sich in diesen hinein erstreckendes Sackloch 38 auf, welches auf der proximalen Seite mit einem Boden 39 verschlossen ist. Die Außenseite des Kolbenstopfens 16 liegt dichtend, jedoch gleitverschieblich an der Innenseite des Spritzenzylinders 15 an.

Der Kupplungsteil 37 umfasst einen Basiskörper 40, von welchem sich ausgehend hin zum Kolbenstopfen 16 - also in proximaler Richtung - ein vorragender Kupplungsfortsatz 41 erstreckt. Der Kupplungsfortsatz 41 ragt mit seinem Kupplungsfortsatzende 42 in das Sackloch 38 hinein. In der unbetätigten Ausgangsstellung der Auspresseinheit 25 ist das Kupplungsfortsatzende 42 bevorzugt distanziert vom Boden 39 angeordnet, sodass der Boden 39 eine noch nicht durchstochene Membran ausbildet, welche bakteriendicht im Zusammenwirken des Kolbenstopfens 16 den Innenraum des Spritzenzylinders 15 verschließt. Weiters ist in der unbetätigten Ausgangsstellung der Kolbenstopfen 16 in axialer Richtung vom Basiskörper 40 distanziert angeordnet. Zur Lagearretierung des Kolbenstopfens 16 am Kupplungsfortsatz 41 kann am Kupplungsfortsatz 41 zumindest ein erstes Rastelement 43 angeordnet bzw. ausgebildet sein, welches in der unbetätigten Ausgangsstellung in eine im Kolbenstopfen 16 im Bereich des Sackloches 38 angeordnete Rastausnehmung 44 eingreift bzw. eingesetzt ist. Dabei wäre es unabhängig davon auch möglich, das Rastelement 43 am Kolbenstopfen 16 und die Rastausnehmung 44 am Kupplungsfortsatz 41 anzuordnen bzw. daran auszubilden.

Durch das Zusammenwirken des ersten Rastelements 43 mit der Rastausnehmung 44 erfolgt eine axiale gegenseitige Halterung bzw. Arretierung des Kolbenstopfens 16 am Kupplungsfortsatz 41. So kann in dieser Stellung die vormontierte Auspresseinheit 25 in den Spritzenzylinder 15 nach Befüllung mit dem abzugebenden Medikament eingesetzt werden. Vor der Befüllung mit dem Medikament ist noch die Einheit des Injektionsspritzenkopfes 21 am Spritzenzylinder 15 zu montieren bzw. zu befestigen. Damit kann zuerst die Einheit des Injektionsspritzenkopfes 21 zusammengebaut werden und ausgehend vom distalen Ende in den Spritzenzylinder 15 eingeschoben werden. Dabei kann die Einheit des Injektionsspritzenkopfes 21 bereits sterilisiert vorliegen. Nach dem Einsetzen in steriler Umgebungsbedingung ist dann das Medikament einzufüllen. Anschließend wird die ebenfalls sterilisierte Auspresseinheit 25 ebenfalls eingesetzt. So ist der das Medikament aufnehmende Innenraum bis hin zur Aktivierung steril. Erst durch die Aktivierung wird ein Zugang zum Medikament über die Kanüle 3 geschaffen.

Das Kupplungsfortsatzende 42 des Kupplungsfortsatzes 41 ist auf der von der Kolbenstange 17 abgewendeten Seite - also an seinem proximalen Ende - pfeilförmig verjüngend ausgebildet. Diese pfeilförmige Verjüngung dient dazu, um in ein im Dichtelement 7 ausgebildetes weiteres Sackloch eintreten zu können und zwischen dem Kupplungsfortsatzende 42 und dem Dichtelement 7 eine Kupplungsverbindung für die Rückzugsbewegung der gesamten Einheit des Injektionsspritzenkopfes 21 zu schaffen. Zur Ausbildung dieser Kupplungsverbindung sind am Kupplungsfortsatzende 42 bevorzugt zumindest zwei den Querschnitt des Kupplungsfortsatzes 41 überragende Rastarme 45 vorgesehen. Der oder die Rastarme 45 weisen eine gewisse Eigensteifigkeit auf und können durch die elastische Verformung des Werkstoffs des Dichtelements 7 in das weitere Sackloch eingeschoben werden. Durch diese Ausbildung der Rastarme 45 ist das Eintreten des Kupplungsfortsatzendes 42 in das Sackloch des Dichtelements 7 einfacher und ohne größeren Widerstand möglich, wobei nach einem Einschubweg der oder die Rastarme 45 hinterhalb einer im Sackloch des Dichtelements 7 angeordneten bzw. ausgebildeten Schulter angeordnet sind. Je nach Stärke und gewähltem Werkstoff des Rastarms 45 kann dieser in gewissem Umfang elastische Eigenschaften aufweisen. Aufgrund des geringen Querschnitts des Rastarms 45 kann dieser mit der Schulter im Sackloch des Dichtelements 7 die gewünschte Kupplungsverbindung bilden. Dadurch wird eine gewisse Verankerung bzw. Verrastung des oder der Rastarme 45 im elastisch verformbaren Dichtelement 7 bei der Rückstellbewegung erzielt. Gerade bei silikonisierten Oberflächen des Dichtelements 7 ist diese Ausbildung der Rastarme 45 anstelle eines zumeist umlaufend ausgebildeten Rastkegels vorteilhaft. So kann die Schulter und die in distaler Richtung vorgeordnete Durchtrittsöffnung mit einem geringeren Querschnitt ausgebildet werden, der lediglich der Aufnahme des Querschnitts des Kupplungsfortsatzes 41 dient. Der oder die darüber hinaus vorragenden Rastarme 45 mit der geringen Querschnittsbreite bezogen auf den gesamten Umfang des Kupplungsfortsatzes 41 dringen dann bedingt durch die elastische Verformung des Dichtelements 7 sowie gegebenenfalls einer radialen elastischen Verformung des oder der Rastarme 45 in Verbindung mit einer Freistellung im Kupplungsfortsatz 41 in das Sackloch ein.

Zur Kupplung des Kupplungsteils 37 mit der Kolbenstange 17 können am Basiskörper 40 des Kupplungsteils 37 auf der der Kolbenstange 17 zugewendeten Seite elastisch verformbare Kupplungsarme 46 vorgesehen sein, die mit einem an der Kolbenstange 17 ausgebildeten Kupplungselement 47 lösbar gekuppelt werden können. Dabei sind die Kupplungsarme 46 bevorzugt in radialer Richtung federnd ausgebildet und hintergreifen das Kupplungselement 47 auf der der Kolbenstange 17 zugewendeten Seite. Die Dimensionen und Abmessungen der Kupplungsarme 46 in der gekuppelten Stellung mit dem Kupplungselement 47 sind so gewählt, dass diese eine Außenabmessung aufweisen, welche in etwa der Innenabmessung des Spritzenzylinders 15 entsprechen. Damit ist ein Entkuppeln der Kolbenstange 17 solange nicht möglich bis dass eine gewisse Querschnittserweiterung erfolgt, um die radiale Aufweitung der Kupplungselemente 46 bewirken zu können und so den Entkupplungsvorgang durchzuführen. Da der Spritzenzylinder 15 zumeist aus Glas gebildet ist, ist es vorteilhaft, wenn seine Innenwandung über dessen gesamte Länge nahezu den gleichen Querschnitt bzw. die gleiche Querschnittsabmessung aufweist.

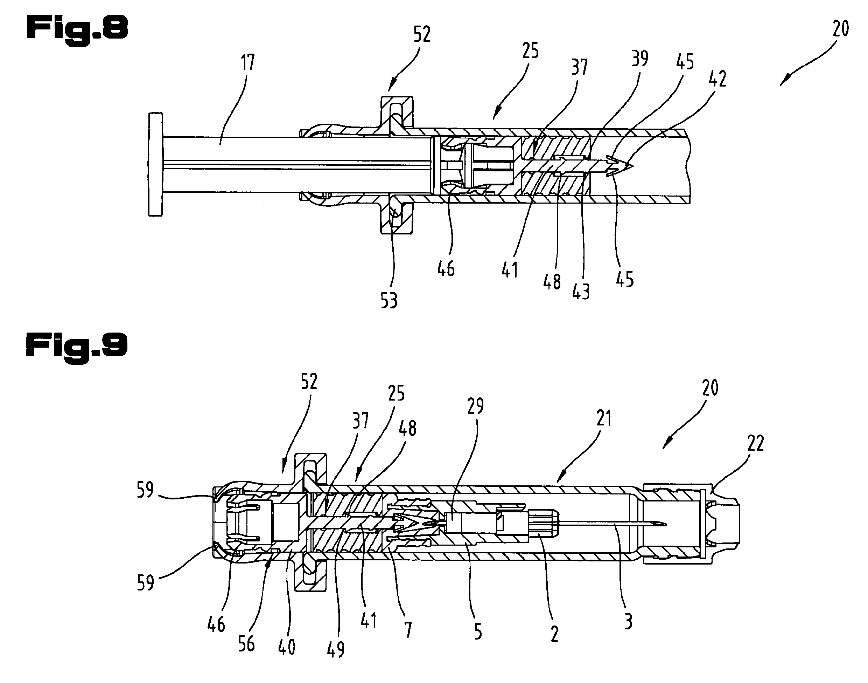

Wie nun besser aus der Fig. 8 zu ersehen ist, befindet sich hier die Auspresseinheit 25 in ihrer aktivierten Stellung, bei welcher das Kupplungsfortsatzende 42 den Boden 39 des Kolbenstopfens 16 durchstochen hat und mit seinem distalen Ende am proximalen Ende des Basiskörpers 40 anliegt bzw. abgestützt ist. In dieser Auspressstellung liegt das erste Rastelement 43, welches zuvor in der Rastausnehmung 44 des Kolbenstopfens 16 angeordnet war, nach der relativen Verlagerung an der Innenseite des Bodens 39 des Sackloches 38 in dem vom Kupplungsfortsatz 41 durchsetzten Bereich an. Durch diese Abstützwirkung des Bodens 39 wird eine zusätzliche Dichtwirkung an der proximalen Stirnseite des Kolbenstopfens im Kontaktbereich bzw. Durchtrittsbereich des Kupplungsfortsatzes 41 geschaffen. Dies deshalb, da der Boden 39 eine relativ dünne Wandstärke aufweist und so der während des Abgabevorgangs auftretende innere Druck in diesem Bereich zu keiner Undichtheit führt.

In der Fig. 9 ist die zurückgezogene Stellung und somit die Schutzstellung der Nadeleinheit 12 innerhalb des Spritzenzylinders 15 dargestellt. Dabei ist die Kolbenstange 17 bereits vom Kupplungsteil 37 getrennt worden. Um diese Rückzugsbewegung des Kolbenstopfens 16 besser durchführen zu können, kann zusätzlich am Kupplungsfortsatz 41 zwischen dem Basiskörper 40 und dem ersten Rastelement 43 zumindest ein weiteres Rastelement 48 angeordnet bzw. ausgebildet sein. Dieses liegt bei der Rückstellbewegung der Auspresseinheit 25 mitsamt der Nadeleinheit 12 an einer im Sackloch 38 des Kolbenstopfens 16 ausgebildeten Schulter 49 an. Zur Ausbildung der Schulter 39 kann das Sackloch Abschnitte mit unterschiedlichen Querschnitten 50, 51 aufweisen, wobei beim hier gezeigten Ausführungsbeispiel der Querschnitt 51 eine kleinere Querschnittsabmessung aufweist als der Querschnitt 50 im Bereich des dem Boden 39 zugewendeten Abschnitts.

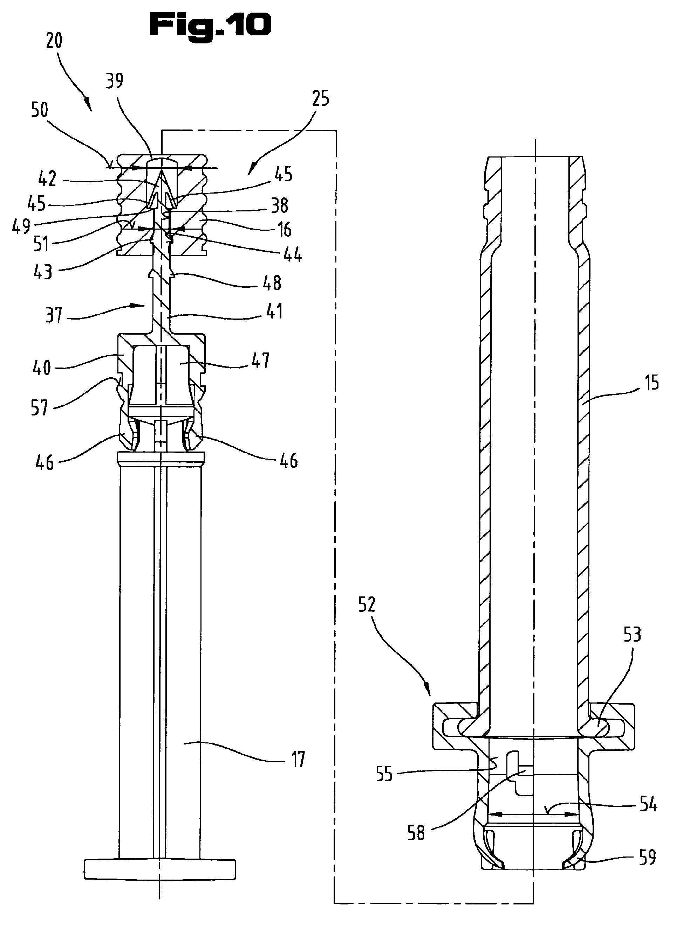

Wie nun besser aus einer Zusammenschau der Fig. 9 und 10 zu ersehen ist, weist, wie bereits zuvor beschrieben, der Spritzenzylinder 15 ausgehend von seinem verengten proximalen Endabschnitt hin zum distalen Ende eine nahezu gleiche Querschnittsabmessung auf. Am distalen Ende des Spritzenzylinders 15 ist weiters noch vereinfacht ein Handhabungselement 52 gezeigt, welches bevorzugt Bestandteil der Auspresseinheit 25 ist. Dieses Handhabungselement 52 wird bevorzugt aus zwei in radialer Richtung zusammenfügbaren Halbkörpern gebildet, welche über einen Fortsatz 53 des Spritzenzylinders 15 an diesem in axialer Richtung festgelegt gehalten sind. Für die gegenseitige Verbindung der beiden Halbkörper zur Bildung des Handhabungselements 52 können jegliche bekannte Schnapp- und/oder Rastelemente dienen.

Um den zuvor beschriebenen Entkupplungsvorgang zwischen dem Kupplungselement 47 der Kolbenstange 17 und den Kupplungsarmen 46 des Kupplungsteils 37 durchführen zu können, ist eine lichte innere Weite 54 eines Durchtrittskanals 55 im Handhabungselement 52 in einem distalen Abschnitt größer als eine äußere Querschnittsabmessung der Kupplungsarme 46 in der unverformten Stellung. Damit wird eine radiale Aufweitung der Kupplungsarme 46 ermöglicht, um so den Entkupplungsvorgang durchführen zu können.

Zur Erzielung einer axialen Lagefixierung des Kupplungsteils 47 relativ bezüglich des Spritzenzylinders 15 und damit eine erneute Wiederverwendung der Injektionsspritze 20 zu vermeiden, ist es vorteilhaft, wenn zwischen dem Kupplungsteil 37, insbesondere des Basiskörpers 40, und dem Handhabungselement 52 eine Haltevorrichtung 56 mit zusammenwirkenden Halteelementen 57, 58 ausgebildet ist. Bei diesem hier gezeigten Ausführungsbeispiel ist das erste Halteelement 57 am Basisteil 40 des Kupplungsteils 37 beispielsweise in Form einer umlaufenden nutförmigen Vertiefung ausgebildet, in welche zumindest das weitere Halteelement 58 in der Sicherungsstellung rastend eingreift. Das weitere Halteelement 58 im Bereich des Handhabungselements 52 kann radial federnd bzw. elastisch verformbar ausgebildet sein, welches nach ausreichender Rückstellbewegung des Kupplungsteils 37 in das dafür vorgesehene erste Halteelement 57 arretierend einrastet. Je nach Ausbildung und Art der gewählten Halteelemente 57, 58 zueinander kann eine axiale Verlagerung der verbliebenen Auspresseinheit 25 sowie der Nadeleinheit 12 in zumindest eine Richtung - proximal und/oder distal - verhindert werden. Bevorzugt kann damit aber auch ein weiteres Herausziehen der verbliebenen Auspresseinheit 25 mitsamt der Nadeleinheit 12 verhindert werden.

Um ein erneutes Einsetzen der Kolbenstange 17 mit dessen Kupplungselement 47 in den Kupplungsteil 37 insbesondere dessen Basiskörper 40 mit den Kupplungsarmen 46 zu verhindern, kann am Handhabungselement 52 im Bereich seines distalen Endes zumindest ein Leitelement 59 angeordnet sein, welches in den Querschnitt des Durchtrittskanals 55 hineinragt. Das Leitelement 59 kann beispielsweise bogenförmig gekrümmt hin in Richtung auf die Längsachse ausgebildet sein. Bevorzugt werden jedoch mehrere Leitelemente 59 vorgesehen, um so das erneuet Wiedereinsetzen der Kolbenstange 17 in die Injektionsspritze 20 zu verhindern.

Es versteht sich, dass die geschilderten Ausführungsbeispiele im Rahmen des Erfindungsgedankens verschiedentlich abwandelbar sind, z.B. hinsichtlich der Anordnung der Führungsnoppen 1 relativ zueinander und/oder relativ zum Rasthaken 8, hinsichtlich der Materialen sowie dem Steigungswinkel der Kulissenbahnen 10.

Bei den zuvor beschrieben Injektionsspritzen 20 handelt es sich um einen Spritzentyp, bei welchem bereits das Medikament eingefüllt und für die Abgabe bereitgestellt ist. Dabei spricht man von einer Fertigspritze, insbesondere Glasfertigspritze. Der Werkstoff des Spritzenzylinders 15 ist aus Glas gewählt. Der das Medikament aufnehmende Spritzeninnenraum wird an seinem proximalen Ende vollständig durch das bis hin zur Aktivierung undurchstochene Dichtelement 7 und an seinem distalen Ende durch den ebenfalls noch undurchstochenen Kolbenstopfen 16 gegen die Innenwand des Spritzenzylinder 15 bakteriendicht abgedichtet. Erst nach der Aktivierung des Injektionsspritzenkopfes 21 durch Verdrehen der Schutzkappenspitze 23 durchsticht das distale Ende der Kanüle 3 den Boden des Dichtelements 7 und stellt somit die Leitungsverbindung mit dem Innenraum des Spritzenzylinders 15 und damit verbunden dem Medikament her. Dann kann die Abgabe des bevorrateten Medikaments erfolgen.

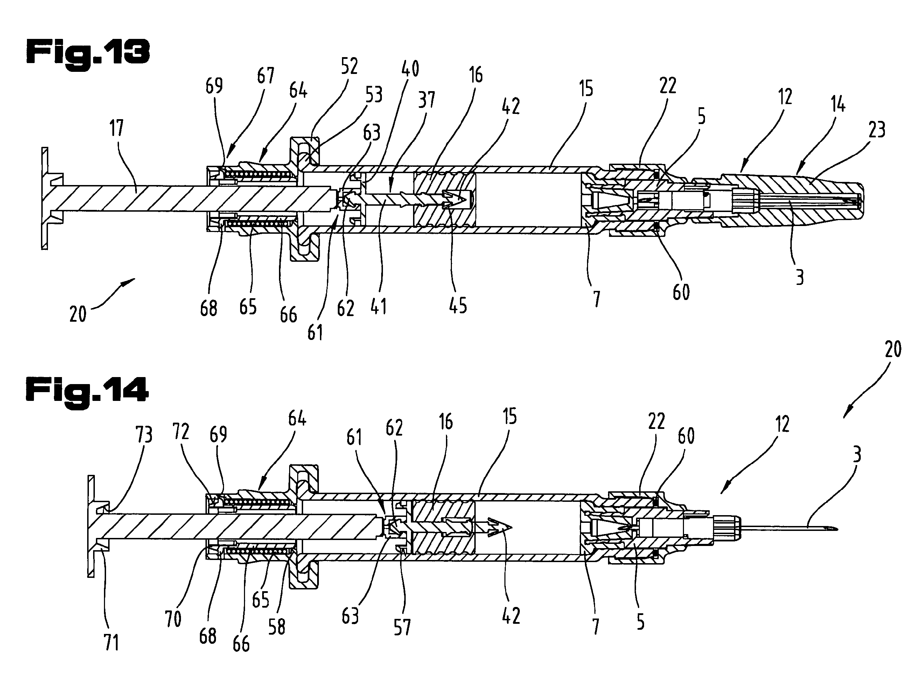

In den Fig. 13 bis 16 ist eine weitere und gegebenenfalls für sich eigenständige Ausführungsform einer Injektionsspritze 20 gezeigt, wobei wiederum für gleiche Teile gleiche Bezugszeichen bzw. Bauteilbezeichnungen wie in den vorangegangenen Fig. 1 bis 12 verwendet werden. Um unnötige Wiederholungen zu vermeiden, wird auf die detaillierte Beschreibung in den vorangegangenen Fig. 1 bis 12 hingewiesen bzw. Bezug genommen.

Diese hier gezeigte Injektionsspritze 20 ist dabei ähnlich ausgebildet, wie diese bereits zuvor, insbesondere in den Fig. 6 bis 12, beschrieben worden ist. Bei dieser Ausführungsvariante soll es dem Benutzer bzw. Anwender einer derartigen Injektionsspritze 20 möglich sein, dass nach erfolgter Abgabe des innerhalb des Spritzenzylinders 15 bevorrateten Medikaments ohne weiteres Zutun des Benutzers eine Schutzstellung der gebrauchten Nadel erreichbar ist. Erfolgt diese ohne Zutun des Benutzers, kann dabei von einem passiven Nadelschutzsystem gesprochen werden. Sind hingegen vom Benutzer eigene Schritte nach erfolgter Abgabe des Medikaments aus dem Spritzenzylinder zu setzen, dass eine Nadelschutzstellung erzielt wird, wird von einem aktiven Nadelschutzsystem gesprochen. Dieser eigene Schritt kann eine bewusst und gezielte Aktivierung eines Mechanismus sein

So umfasst die hier dargestellte Injektionsspritze 20 den Spritzenzylinder 15 zur Aufnahme des nicht näher dargestellten Medikaments, die Kolbenstange 17 mit dem damit gekuppelten Kupplungsteil 37, an welchem auch noch der Kolbenstopfen 16 angeordnet sein kann. Die Nadeleinheit 12 ist wiederum derart ausgebildet, dass diese von der Schutzkappe 14 in der unbenutzten Stellung abgedeckt ist und das distale Ende der Kanüle 3 noch nicht mit dem Innenraum des Spritzenzylinders 15 in Strömungsverbindung steht. Zur Herstellung der Strömungsverbindung ist das distale Ende der Kanüle 3 durch das Dichtelement 7 hindurchzustechen, wodurch die gewünschte Strömungsverbindung hergestellt werden kann. Die Schutzkappe 14 kann ihrerseits wiederum die Schutzkappenbasis 22 sowie die damit im Auslieferungszustand verbundene Schutzkappenspitze 23 umfassen. Mit dieser ist die Aktivierung der Kanüle 3 durchzuführen, wie dies bereits zuvor beschrieben worden ist.

Der Kupplungsteil 37 dient auch hier wiederum dazu den Kolbenstopfen 16 und die Kolbenstange 17 miteinander zu kuppeln bzw. zu verbinden. Dazu ist am Basiskörper 40 des Kupplungsteils 37 auf der der Kolbenstange 17 zugewendeten Seite eine eigene Kupplungsvorrichtung 61 vorgesehen, wobei die Kupplungsvorrichtung 61 zusammenwirkende Kupplungselemente 62, 63 umfassen kann. Das hier dargestellte erste Kupplungselement 62 kann beispielsweise als kegelstumpfförmiger Ansatz ausgebildet sein, welcher auf seiner dem Basiskörper 40 zugewendeten Seite mit einer Hinterschneidung versehen ist und sich in axialer Richtung erstreckt. Am proximalen Ende der Kolbenstange 17 ist das bzw. sind die weiteren Kupplungselemente 63 angeordnet, welche in der Kupplungsstellung mit dem ersten Kupplungselement 62 in Kupplungseingriff stehen.

Weiters ist am distalen Ende des Spritzenzylinders 15 das zuvor beschriebene Handhabungselement 52 angeordnet, welches in dessen Zentrum von der Kolbenstange 17 durchragt ist. Das Handhabungselement 52 ist bevorzugt am Fortsatz 53 des Spritzenzylinders 15 gehaltert.

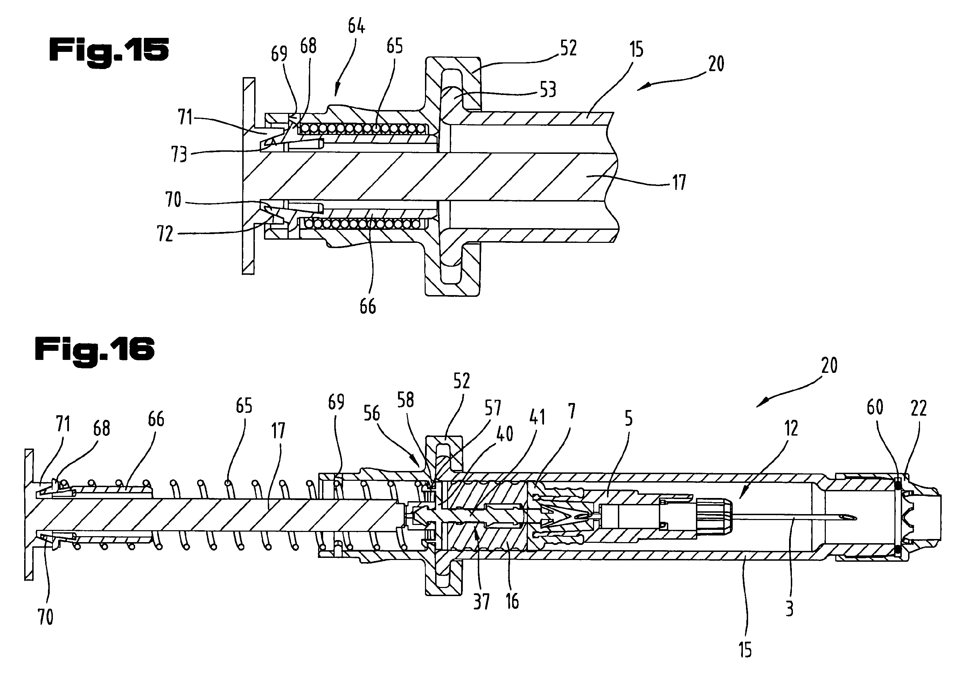

Zwischen dem Handhabungselement 52 und der Kolbenstange 17 ist hier eine Rückstellvorrichtung 64 vorgesehen. Unter "zwischen" wird hier verstanden, dass die Rückstellvorrichtung 64 sich einerseits am Handhabungselement 52 abstützt und andererseits nach deren Freigabe die Kolbenstange 17, der Kupplungsteil 37, der Kolbenstopfen 16 sowie die damit bedarfsweise kuppelbare Nadeleinheit 12 in distaler Richtung relativ bezüglich des Spritzenzylinders 15 von der proximalen Stellung hin in die distale Stellung verlagert werden. Diese zurückverlagerte Stellung ist in der Fig. 16 dargestellt.

So kann beispielsweise die Rückstellvorrichtung 64 zumindest ein Federelement 65 umfassen, welches in der vorgespannten Position bzw. Lage eine in axialer Richtung wirkende Verstellkraft aufbaut, welche nach dem Entriegeln bzw. Lösen der Rückstellvorrichtung 64 wirksam wird. Das Federelement 65 kann eine Druckfeder, insbesondere eine Spiralfeder sein. So ist hier das proximale Ende des Federelements 65 direkt am Handhabungselement 52 abgestützt. Weiters ist hier noch in radialer Richtung gesehen, zwischen dem Federelement 65 und der Kolbenstange 17 ein eigener, insbesondere rohrförmig ausgebildeter Gleitkörper 66 angeordnet. Auf dessen äußeren Umfang bzw. um diesen herum ist das Federelement 65 angeordnet. Der Gleitkörper 66 dient dem Federelement 65 zur Abstützung mit seinem distalen Ende. Weiters ist der Gleitkörper 66 in der vorgespannten Stellung des Federelements 65 am Handhabungselement 52 im Bereich von dessen distalen Ende mittels einer Rückhaltevorrichtung 67 bedarfsweise lösbar gehaltert bzw. abgestützt.

Die Rückhaltevorrichtung 67 kann ihrerseits zusammenwirkende Rückhalteelemente 68, 69 umfassen, wobei hier beispielsweise das Rückhalteelement 68 als nasenförmiger Ansatz am Gleitkörper 66 angeordnet ist, welcher den Gleitkörper 66 in radialer Richtung überragt. Das oder die Rückhalteelemente 68 können an einem freigestellten Federarm angeordnet sein, wodurch das oder die Rückhalteelemente 68 in radialer Richtung hin in Richtung auf die Längsachse verschwenkt werden. Das damit zusammenwirkende, weitere Rückhalteelement 69 kann beispielsweise eine Ausnehmung, ein Schlitz oder ein Durchbruch im Handhabungselement 52 sein, an welchem sich das erste Rückhalteelement 68 unter Abtragung der Federkraft des Federelements 65 daran abstützt.

Damit kann ein vorgespannter Federspeicher geschaffen werden, welcher bei Betätigung bzw. Auslösung die automatisierte Rückstellung und damit den passiven Nadelrückzug bewirkt.

Die Injektionsspritze 20 wird, wie bereits zuvor beschrieben, vorbefüllt mit dem Medikament gelagert und so ausgeliefert, wobei die Betätigung für den Abgabevorgang derart erfolgen kann, wie dies bereits zuvor in den Fig. 1 bis 12 beschrieben worden ist. Bei den Ausführungsformen gemäß der Fig. 1 bis 12 ist es jedoch notwendig gewesen, dass über eine aktive Nadelrückstellung der Benutzer zur Erzielung der Schutzstellung die Nadel innerhalb des Spritzenzylinders 15 selbst zurückziehen musste.

Wie nun besser aus der Fig. 15 zu ersehen ist, wurde hier bereits eine ausreichende Menge des Medikaments aus dem Spritzenzylinder 15 abgegeben, wobei das Kupplungsfortsatzende 42 des Kupplungsteils 37 in Kupplungseingriff mit dem Dichtelement 7 sowie der damit gekuppelten Nadeleinheit 12 gebracht worden ist.

Die Freigabe der Rückhaltevorrichtung 67 erfolgt hier durch zusammenwirkende Stellelemente 70, 71. Diese Stellelemente 70, 71 sind derart auszubilden, dass nach einem vorbestimmten Verstellweg der Kolbenstange 17 relativ bezüglich des Spritzenzylinders 15 bzw. des daran angeordneten Handhabungselements 52 die zusammenwirkenden Rückhalteelemente 68, 69 der Rückhaltevorrichtung 67 soweit außer Eingriff gebracht werden, dass die vorgespannte Rückstellvorrichtung 67 mit ihrem Federelement 65 die Rückstellkraft auswirkt, wodurch die Kolbenstange 17 mitsamt den damit gekuppelten Bauteilen bzw. Elementen soweit zurückstellt werden kann, dass zumindest die Nadeleinheit 12 mit ihrem proximalen Ende der Kanüle 3 sich innerhalb des Spritzenzylinders 15 bzw. der daran gehalterten Schutzkappenbasis 22 befindet.

Die Stellelemente 70, 71 können beispielsweise konisch bzw. winkelig bezüglich der Längsachse der Injektionsspritze 20 verlaufend ausgerichtete Stellflächen 72, 73 aufweisen. Bedingt durch die gleichgleich konische Ausrichtung bzw. winkelige Neigung der Stellflächen 72, 73 kommen diese - wie in Fig. 15 zu ersehen ist - aneinander zur Anlage, wodurch die zuvor beschriebenen Rückhalteelemente 68, 69 außer Rasteingriff miteinander gebracht werden. Die geneigten Stellflächen 72, 73 bewirken bei einem gegenseitigen Aneinanderliegen, dass das hier nasenförmig ausgebildete Rückhalteelement 68 in radialer Richtung hin in Richtung auf die Längsachse verlagert wird und damit außer Eingriff mit dem weiteren Rückhalteelement 69 kommt. Sind diese beiden Rückhaltelemente 68, 69 außer Eingriff gebracht worden, kann das Federelement 65 der Rückstellvorrichtung 64 unter Anlage des Gleitkörpers 66 an der Kolbenstange 70 in deren distalen Ende die Kolbenstange 17 mitsamt den damit gekuppelten Bauteilen zurückverstellen. Diese Schutzstellung der Nadeleinheit 12 ist aus der Fig. 16 zu ersehen. So ist hier beispielsweise das erste Stellelement 70 am Gleitkörper 66 und das weitere Stellelement 71 an der Kolbenstange 17 angeordnet bzw. vorgesehen. So erfolgt die Freigabe der Rückhalteelemente 68, 69 automatisch und selbsttätig im Zuge des Abgabevorganges und es ist kein weiterer gesonderter Auslösevorgang durchzuführen.

Um eine erneute Betätigung des Injektionsspritze 20 zu vermeiden, kann wiederum zwischen dem Kupplungsteil 37, insbesondere dessen Basiskörper 40, und dem Handhabungselement 52 die zuvor beschriebene Haltevorrichtung 56 mit ihren Halteelementen 57, 58 vorgesehen sein. Dabei können die Halteelemente 57, 58 beispielsweise durch zusammenwirkende Rasthaken bzw. Rastnasen gebildet sein, welche derart ineinander greifen, dass in deren Eingriffsstellung eine erneute Betätigung und damit relative Verlagerung der Nadeleinheit 12 bezüglich des Spritzenzylinders 15 gesichert vermieden ist.

In dieser geschützten Stellung der Kanülenspitze der Kanüle 3 innerhalb des Spritzenzylinders 15 kann dann ohne Bedenken eine Entsorgung der gesamte Injektionsspritze 20 erfolgen.

Damit kann erreicht werden, dass durch diese Ausbildung der Rückstellvorrichtung 64 der Benutzer keine zusätzlichen Auslöseschritte setzen muss, um die Schutzstellung der Kanüle 3 zu erzielen, sondern dies im Zuge des passiven Nadelrückzugs durch die Rückstellungsvorrichtung 64 automatisiert erfolgt.

Die zuvor beschriebene Rückstellvorrichtung 67 kann aber auch unabhängig davon mit einer Injektionsspritze kombiniert werden, wie diese beispielsweise in der WO 2007/112470 A1 beschrieben worden ist. Diese Injektionsspritze unterscheidet sich zu der hier vorliegenden Ausführungsform nur dadurch, dass die Kanüle bereits von Anbeginn an mit dem Innenraum des Spritzenzylinders und damit mit dem Medikament in Strömungsverbindung steht. Bei dieser hier in den Fig. 1 bis 16 beschriebenen Ausführungsform wird das distale Ende der Kanüle 3 erst unmittelbar kurz bevor der beabsichtigten Abgabe des Medikaments in Strömungsverbindung mit dem Innenraum des Spritzenzylinders 15 und damit dem darin bevorrateten Medikament gebracht.

Weiters wird die unbenutzte Stellung der einzelnen Bauteile zueinander als Ruhestellung bzw. Originalitätszustand und jene Stellung der Nadeleinheit 12, bei welcher eine Leitungsverbindung mit dem Innenraum des Spritzenzylinders 15 hergestellt worden ist, als Aktivierungsstellung bezeichnet. Gleiche Betriebszustände bzw. Positionen der Auspresseinheit 25 können ebenfalls mit diesen Angaben bezeichnet werden. Ist die Nadeleinheit 12 in den Spritzenzylinder 15 zurückverstellt worden, kann von einer Entsorgungs- bzw. Schutzstellung gesprochen werden.

Der Ordnung halber sei abschließend darauf hingewiesen, dass zum besseren Verständnis des Aufbaus der Injektionsspritze 20, insbesondere deren Injektionsspritzenkopf 21 sowie deren Auspresseinheit 25, diese bzw. deren Bestandteile teilweise unmaßstäblich und/oder vergrößert und/oder verkleinert dargestellt wurden.

Die den eigenständigen erfinderischen Lösungen zugrundeliegende Aufgabe kann der Beschreibung entnommen werden.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mitumfasst sind, d.h. sämtliche Teilbereich beginnen mit einer unteren Grenze von 1 oder größer und enden bei einer oberen Grenze von 10 oder weniger, z.B. 1 bis 1,7, oder 3,2 bis 8,1 oder 5,5 bis 10.

Die Ausführungsbeispiele zeigen mögliche Ausführungsvarianten der Injektionsspritze 20, insbesondere deren Injektionsspritzenkopf 21 sowie deren Auspresseinheit 25, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schutzumfang mit umfasst. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Vor allem können die einzelnen in den Fig. 1, 2, 3; 4; 5; 6 bis 12; 13 bis 16 gezeigten Ausführungen den Gegenstand von eigenständigen, erfindungsgemäßen Lösungen bilden. Die diesbezüglichen, erfindungsgemäßen Aufgaben und Lösungen sind den Detailbeschreibungen dieser Figuren zu entnehmen.

**Bezugszeichenaufstellung**

| | | | |
|---|---|---|---|
| 1 | Führungsnoppe | 31 | Längsrippe |
| 2 | Triebling | 32 | Rastorgan |
| 3 | Kanüle | 33 | Arretierelement |
| 4 | Kanülenhalter | 34 | Arretierelement |
| 5 | Führungshülse | 35 | Arretiervorrichtung |
| | | | |
| 6 | Kupplung | 36 | Bodenelement |
| 7 | Dichtelement | 37 | Kupplungsteil |
| 8 | Rasthaken | 38 | Sackloch |
| 9 | Fenster | 39 | Boden |
| 10 | Kulissenbahn | 40 | Basiskörper |
| | | | |
| 11 | Kulissenerweiterung | 41 | Kupplungsfortsatz |
| 12 | Nadeleinheit | 42 | Kupplungsfortsatzende |
| 13 | Steg | 43 | Rastelement |
| 14 | Schutzkappe | 44 | Rastausnehmung |
| 15 | Spritzenzylinder | 45 | Rastarm |
| | | | |
| 16 | Kolbenstopfen | 46 | Kupplungsarm |
| 17 | Kolbenstange | 47 | Kupplungselement |
| 18 | Freistellung | 48 | Rastelement |
| 19 | Dichtscheibe | 49 | Schulter |
| 20 | Injektionsspritze | 50 | Querschnitt |
| | | | |
| 21 | Injektionsspritzenkopf | 51 | Querschnitt |
| 22 | Schutzkappenbasis | 52 | Handhabungselement |
| 23 | Schutzkappenspitze | 53 | Fortsatz |
| 24 | Sollbruchstelle | 54 | lichte Weite |
| 25 | Auspresseinheit | 55 | Durchtrittskanal |
| | | | |
| 26 | Vorsprung | 56 | Haltevorrichtung |
| 27 | Ausnehmung | 57 | Halteelement |
| 28 | Schaft | 58 | Halteelement |
| 29 | Ansatz | 59 | Leitelement |
| 30 | Führungsorgan | 60 | Dichtring |
| 61 | Kupplungsvorrichtung | | |
| 62 | Kupplungselement | | |
| 63 | Kupplungselement | | |
| 64 | Rückstellvorrichtung | | |
| 65 | Federelement | | |
| 66 | Gleitkörper | | |
| | | | |
| 67 | Rückhaltevorrichtung | | |
| 68 | Rückhalteelement | | |
| 69 | Rückhalteelement | | |
| 70 | Stellelement | | |
| | | | |
| 71 | Stellelement | | |
| 72 | Stellfläche | | |
| 73 | Stellfläche | | |

## Patentansprüche

1. Injektionsspritze (20) umfassend einen Spritzenzylinder (15) zur Aufnahme eines abzugebenden Medikaments, eine am distalen Ende des Spritzenzylinders (15) angeordnete Auspresseinheit (25), einen am proximalen Ende des Spritzenzylinders (15) angeordneten Injektionsspritzenkopf (21) mit einer in einer Führungshülse (5) angeordneten und darin gleitverschieblichen Nadeleinheit (12) mit einer Kanüle (3) und einem Kanülenhalter (4), und einem Triebling (2), durch den die Nadeleinheit (12) axial relativ zum Spritzenzylinder (15) von einer Ruhestellung hin zu einer Aktivierungsstellung bewegbar ist, und einem am distalen Ende der Führungshülse (5) angeordneten Dichtelement (7), welches in der Ruhestellung der Nadeleinheit (12) von der Kanüle (3) undurchstochen und in der Aktivierungsstellung der Nadeleinheit (12) von der Kanüle (3) durchstochen ist, **dadurch gekennzeichnet, dass** der Triebling (2) zumindest eine Führungsnoppe (1) aufweist, die wahlweise mit einer von zwei in der Führungshülse (5) vorgesehenen Kulissenbahnen (10) in Eingriff versetzbar ist, wobei die Kulissenbahnen (10) gegensinnige Steigungen aufweisen.

2. Injektionsspritze (20) nach Anspruch 1, **dadurch gekennzeichnet, dass** der Triebling (2) zwei Führungsnoppen (1) umfasst, welche in einer senkrecht zu einer Längsachse ausgerichteten Ebene sowie in Umfangsrichtung voneinander distanziert angeordnet sind und zur Verlagerung der Nadeleinheit (12) von der Ruhestellung hin zur Aktivierungsstellung nur einer der beiden Führungsnoppen (1) mit einer der beiden Kulissenbahnen (10) in Eingriff versetzbar ist.

3. Injektionsspritze (20) nach Anspruch 2, **dadurch gekennzeichnet, dass** in der Führungshülse (5) in einem Umfangsbereich zwischen den beiden Kulissenbahnen (10) eine Freistellung (18) ausgebildet ist und die Freistellung (18) zur Aufnahme jenes Führungsnoppens (1) dient, welcher während der Verstellung der Nadeleinheit (12) mit keiner der Kulissenbahnen (10) in Eingriff steht.

4. Injektionsspritze (20) nach einem der Ansprüche1 bis 3, **dadurch gekennzeichnet, dass** die Nadeleinheit (12), insbesondere deren Kanülenhalter (4), und die Führungshülse (5) in der Aktivierungsstellung mittels zusammenwirkender Arretierelemente (33, 34) einer Arretiervorrichtung (35) in Axialrichtung relativ zueinander positioniert gehalten sind.

5. Injektionsspritze (20) nach einem der Ansprüche 1 bis 4, **dadurch gekennzeichnet, dass** die Nadeleinheit (12), insbesondere deren Kanülenhalter (4), mit dem Triebling (2) um die Längsachse drehbar, jedoch in Axialrichtung arretiert gekuppelt ist.

6. Injektionsspritze (20) nach einem der Ansprüche 1 bis 5, **dadurch gekennzeichnet, dass** die Nadeleinheit (12), insbesondere deren Kanülenhalter (4), in der Führungshülse (5) in Axialrichtung geradlinig geführt gelagert ist.

7. Injektionsspritze (20) nach einem der Ansprüche 1 bis 6, **dadurch gekennzeichnet, dass** am Ende jeder Kulissenbahn (10) eine Kulissenerweiterung (11) vorgesehen ist, in welcher der Führungsnoppen (1) in der Aktivierungsstellung der Nadeleinheit (12) einrastbar ist.

8. Injektionsspritze (20) nach einem der Ansprüche 1 bis 7, **dadurch gekennzeichnet, dass** das distale Ende der Führungshülse (5) als Kupplung (6) ausgebildet ist.

9. Injektionsspritze (20) nach einem der Ansprüche 1 bis 8, **dadurch gekennzeichnet, dass** an einer Außenseite der Führungshülse (5) zumindest eine Längsrippe (31) angeordnet ist, welche verdrehfest an einem Rastorgan (32) einer den Injektionsspritzenkopf (21) aufnehmenden Schutzkappe (14) abgestützt ist.

10. Injektionsspritze (20) nach Anspruch 1, **dadurch gekennzeichnet, dass** die Auspresseinheit (25) eine Kolbenstange (17), einen Kupplungsteil (37) mit einem Basiskörper (40) und einem auf die von der Kolbenstange (17) abgewendete Seite vorragenden Kupplungsfortsatz (41) sowie einen Kolbenstopfen (16) umfasst, wobei der Basiskörper (40) des Kupplungsteils (37) mit der Kolbenstange (17) lösbar gekuppelt ist, und der Kolbenstopfen (16) ein sich ausgehend von einem der Kolbenstange (17) zugewendeten Ende hinein erstreckendes Sackloch (38) aufweist, das mit einem Boden (39) verschlossen ist, wobei der Kupplungsfortsatz (41) des Kupplungsteils (37) in das Sackloch (38) hineinragt und in einer unbetätigten Ausgansstellung der Kolbenstopfen (16) in axialer Richtung vom Basiskörper (40) distanziert angeordnet ist und weiters der Boden (39) des Sackloches (38) von einem Kupplungsfortsatzende (42) des Kupplungsfortsatzes (41) undurchstochen ist, wobei am Kupplungsfortsatz (41) zumindest ein erstes Rastelement (43) angeordnet ist, welches in der unbetätigten Ausgansstellung in eine im Kolbenstopfen (16) im Bereich des Sackloches (38) angeordnete Rastausnehmung (44) eingesetzt ist.

11. Injektionsspritze (20) nach Anspruch 10, **dadurch gekennzeichnet, dass** das erste Rastelement (43) bei aneinander liegender Stellung des Basiskörpers (40) und des Kolbenstopfens (16) am Boden (39) des Sackloches (38) in dem vom Kupplungsfortsatz (41) durchsetzten Bereich anliegt.

12. Injektionsspritze (20) nach Anspruch 10 oder 11, **dadurch gekennzeichnet, dass** am Kupplungsfortsatz (41) zwischen dem Basiskörper (40) und dem ersten Rastelement (43) ein weiteres Rastelement (48) angeordnet ist, welches bei der Rückstellbewegung der Auspresseinheit (25) an einer im Sackloch (38) des Kolbenstopfens (16) ausgebildeten Schulter (49) anliegend abgestützt ist.

13. Injektionsspritze (20) nach einem der Ansprüche 10 bis 12, **dadurch gekennzeichnet, dass** das Kupplungsfortsatzende (42) auf die von der Kolbenstange (17) abgewendete Seite pfeilförmig verjüngend ausgebildet ist und zumindest einen, bevorzugt zwei, den Querschnitt des Kupplungsfortsatzes (41) überragende Rastarme (45) umfasst.

14. Injektionsspritze (20) nach einem der Ansprüche 10 bis 13, **dadurch gekennzeichnet, dass** der Basiskörper (40) des Kupplungsteils (37) auf der der Kolbenstange (17) zugewendeten Seite elastisch verformbare Kupplungsarme (46) umfasst, die mit einem an der Kolbenstange (17) ausgebildeten Kupplungselement (47) lösbar gekuppelt sind.

15. Injektionsspritze (20) nach Anspruch 14, **dadurch gekennzeichnet, dass** diese weiters noch ein Handhabungselement (52) umfasst, welches mit einem distalen Ende eines Spritzenzylinders (15) kuppelbar ist, und eine lichte innere Weite (54) eines Durchtrittskanals (55) im Handhabungselement (52) in einem distalen Abschnitt größer ausgebildet ist als eine äußere Querschnittsabmessung der Kupplungsarme (46) in deren unverformten Stellung.

16. Injektionsspritze (20) nach Anspruch 15, **dadurch gekennzeichnet, dass** zwischen dem Basiskörper (40) des Kupplungsteils (37) und dem Handhabungselement (52) eine Haltevorrichtung (56) mit zusammenwirkenden Halteelementen (57, 58) ausgebildet ist, welche bei sich in Eingriff befindlichen Halteelementen (57, 58) zumindest eine axiale Verstellung des Kupplungsteils (37) in proximaler Richtung verhindern.

17. Injektionsspritze (20) nach einem der Ansprüche 15 oder 16, **dadurch gekennzeichnet, dass** am Handhabungselement (52) im Bereich seines distalen Endes zumindest ein Leitelement (58) angeordnet ist, welches in den Querschnitt des Durchtrittskanals (55) hineinragt.

18. Injektionsspritze (20) nach einem der Ansprüche 10 bis 17, **dadurch gekennzeichnet, dass** zwischen dem Handhabungselement (52) und der Kolbenstange (17) eine Rückstellvorrichtung (64) vorgesehen ist, mit welcher die Kolbenstange (17), der Kupplungsteil (37), der Kolbenstopfen (16) sowie die damit bedarfsweise kuppelbare Nadeleinheit (12) in distaler Richtung relativ bezüglich des Spritzenzylinders (15) rückverstellbar sind.

19. Injektionsspritze (20) nach Anspruch 18, **dadurch gekennzeichnet, dass** die Rückstellvorrichtung (64) zumindest ein Federelement (65) umfasst, welches mit seinem proximalen Ende am Handhebungselement (52) und mit seinem distalen Ende an einem rohrförmigen Gleitkörper (66) abgestützt ist und der Gleitkörper (66) in der vorgespannten Stellung des Federelements (65) am Handhabungselement (52) mittels einer Rückhaltevorrichtung (67) bedarfsweise lösbar gehalten ist.

20. Injektionsspritze (20) nach Anspruch 19, **dadurch gekennzeichnet, dass** die Freigabe der Rückhaltevorrichtung (67) nach einem vorbestimmten Verstellweg der Kolbenstange (17) relativ bezüglich des Spritzenzylinders (15) durch zusammenwirkende Stellelemente (70, 71) erfolgt, wobei zumindest ein erstes Stellelement (70) am Gleitkörper (66) und zumindest ein weiteres Stellelement (71) an der Kolbenstange (17) angeordnet ist.

21. Injektionsspritze (20) nach einem der vorhergehenden Ansprüche, **dadurch gekennzeichnet, dass** der Werkstoff des Spritzenzylinders (15) aus Glas gewählt ist und im Spritzeninnenraum ein Medikament eingefüllt und für die Abgabe bereitgestellt ist, wobei der das Medikament aufnehmende Spritzeninnenraum an seinem proximalen Ende vollständig durch das bis hin zur Aktivierung undurchstochene Dichtelement (7) und an seinem distalen Ende durch den ebenfalls noch undurchstochenen Kolbenstopfen (16) gegen die Innenwand des Spritzenzylinder (15) bakteriendicht abgedichtet ist.

## Claims

1. Syringe (20) comprising a syringe barrel (15) for holding a medicine to be administered, an ejector unit (25) arranged at a distal end of the syringe barrel (15), a syringe head (21) arranged at a proximal end of the syringe barrel (15) with a needle unit (12) arranged in a guide sleeve (5) and slidable therein comprising a cannula (3) and a cannula holder (4) and a pinion (2), by means of which the needle unit (12) can be moved axially relative to a syringe barrel (15) from a position of rest to an actuation position and a sealing element (7) arranged at the distal end of the guide sleeve (5) which in the position of the rest of the needle unit (12) is unpierced by the cannula (3), and in the actuation position of the needle unit (12) is pierced by the cannula (3), **characterized in that** the pinion (2) comprises at least one guide knob (1), which can be moved into engagement optionally with one of two slide tracks (10) provided in the guide sleeve (5), and the slide tracks (10) are inclined in opposite directions.

2. Syringe (20) as claimed in claim 1, **characterized in that** the pinion (2) comprises two guide knobs (1), which are arranged spaced apart from one another in a plane aligned perpendicular to a longitudinal axis and in circumferential direction and in order to displace the needle unit (12) from the position of rest to the actuation position only one of the two guide knobs (1) can be moved into engagement with one of the two slide tracks (10).

3. Syringe (20) as claimed in claim 2, **characterized in that** in the guide sleeve (5) in a circumferential area between the two slide tracks (10) a free space (18) is formed and the free space (18) is used for receiving that guide knob (1), which during the adjustment of the needle unit (12) is not in engagement with any of the slide tracks (10).

4. Syringe (20) as claimed in one of claims 1 to 3, **characterized in that** the needle unit (12), in particular its cannula holder (4), and the guide sleeve (5) are held in position relative to one another in axial direction in the actuation position by means of cooperating locking elements (33, 34) of a locking device (35).

5. Syringe (20) as claimed in one of claims 1 to 4, **characterized in that** the needle unit (12), in particular its cannula holder (4), is coupled with the pinion (2) to be rotatable about the longitudinal axis but locked in axial direction.

6. Syringe (20) as claimed in one of claims 1 to 5, **characterized in that** the needle unit (12), in particular its cannula holder (4), is mounted in the guide sleeve (5) so as to be guided linearly in axial direction.

7. Syringe (20) as claimed in one of claims 1 to 6, **characterized in that** at the end of each slide track (10) a slide extension (11) is provided in which the guide knob (1) can engage in the actuation position of the needle unit (12).

8. Syringe (20) as claimed in one of claims 1 to 7, **characterized in that** the distal end of the guide sleeve (5) is configured as a coupling (6).

9. Syringe (20) as claimed in one of claims 1 to 8, **characterized in that** on an outer side of the guide sleeve (5) at least one longitudinal rib (31) is arranged, which is supported in a non-rotational manner on a locking element (32) of a protective cap (14) receiving the syringe head (21).

10. Syringe (20) as claimed in claim 1, **characterized in that** the ejector unit (25) comprises a plunger rod (17), a coupling part (37) with a base body (40) and a coupling extension (41) projecting to the side facing away from the plunger rod (17) and a plunger (16), wherein the base body (40) of the coupling part (37) is connected detachably to the plunger rod (17) and the plunger (16) comprises a blind hole (38) extending from an end facing the plunger rod (17), which blind hole (38) is closed by a bottom (39), wherein the coupling extension (41) of the coupling part (37) projects into the blind hole (38) and in an inactivated starting position the plunger (16) is arranged spaced apart in axial direction from the base body (40) and also the bottom (39) of the blind hole (38) is not pierced by a coupling extension end (42) of the coupling extension (41), wherein on the coupling extension (41) at least one first locking element (43) is arranged which in the inactivated starting position is inserted into a locking recess (44) formed in the plunger (16) in the area of the blind hole (38).

11. Syringe (20) as claimed in 10, **characterized in that** the first locking element (43) in an adjoining position of the base body (40) and the plunger (16) rests on the bottom (39) of the blind hole (38) in the area penetrated by the coupling extension (41).

12. Syringe (20) as claimed in claim 10 or 11, **characterized in that** on the coupling extension (41) between the base body (40) and the first locking element (43) a further locking element (48) is arranged, which during the restoring movement of the ejector unit (25) is supported bearing on a shoulder (49) formed in the blind hole (38) of the plunger (16).

13. Syringe (20) as claimed in one of claims 10 to 12, **characterized in that** the coupling extension end (42) is configured to be tapering in an arrow-like manner to the side facing away from the plunger rod (17) and comprises at least one, preferably two, locking arms (45) projecting over the cross section of the coupling extension (41).

14. Syringe (20) as claimed in one of claims 10 to 13, **characterized in that** the base body (40) of the coupling part (37) on the side facing the plunger rod (17) comprises elastically deformable coupling arms (46) which are coupled detachably to a coupling element (47) formed on the plunger rod (17).

15. Syringe (20) as claimed in claim 14, **characterized in that** the latter also comprises an handling element (52), which can be connected to a distal end of a syringe barrels (15), and an internal width (54) of a through channel (55) in the handling element (52) in a distal section is greater than an outer cross-sectional dimension of the coupling arms (46) in their undeformed position.

16. Syringe (20) as claimed in claim 15, **characterized in that** between the base body (40) of the coupling part (37) and the handling element (52) a holding device (56) with interacting holding elements (57, 58) is formed, which when the holding elements (57, 58) are in engagement prevent at least an axial displacement of the coupling part (37) in proximal direction.

17. Syringe (20) as claimed in one of claims 15 or 16, **characterized in that** at least one guiding element (58) is arranged on the handling element (52) in the region of its distal end which projects into the cross section of the through channel (55).

18. Syringe (20) as claimed in one of claims 10 to 17, **characterized in that** between the handling element (52) and the plunger rod (17) a resetting device (64) is provided, by means of which the plunger rod (17), the coupling part (37), the plunger (16) and the needle unit (12) connectable therewith if necessary can be restored in distal direction relative to the syringe barrel (15).

19. Syringe (20) as claimed in claim 18, **characterized in that** the resetting device (64) comprises at least one spring element (65), which is supported at its proximal end on the handling element (52) and at its distal end on a tubular sliding body (66), and the sliding body (66) in the pretensioned position of the spring element (65) is held releasably if necessary on the handling element (52) by means of a retaining device (67).

20. Syringe (20) as claimed in claim 19, **characterized in that** the release of the retaining device (67) is performed after a predetermined adjustment way of the plunger rod (17) relative to the syringe barrel (15) by means of interacting adjusting elements (70, 71), and at least one first adjusting element (70) is arranged on the sliding body (66) and at least one further adjusting element (71) is arranged on the plunger rod (17).

21. Syringe (20) as claimed in one of the preceding claims, **characterized in that** the material of the syringe barrel (15) is selected to be glass and the inner chamber of the syringe is filled with medicine and is ready to be administered, and the inner chamber of the syringe filled with medicine is sealed completely so as to be bacteria-proof from the inner wall of the syringe barrel (15) at its proximal end by the sealing element (7) which is unpierced until activated and at its distal end by the plunger (16) that is also not yet pierced.

## Revendications

1. Seringue d'injection (20) comportant un cylindre d'injection (15) destiné à recevoir un médicament à administrer, une unité d'injection (25) disposée à l'extrémité distale du cylindre d'injection (15), une tête de seringue (21) disposée à l'extrémité proximale du cylindre d'injection (15) et munie d'une unité pour aiguille (12), disposée dans une gaine de guidage (5) de manière mobile en glissement dans celle-ci et munie d'une canule (3) et d'un porte-canule (4), et un poussoir (2), par lequel l'unité pour aiguille (12) peut être déplacée axialement par rapport au cylindre d'injection (15) depuis une position de repos vers une position active, et un élément d'étanchéité (7), qui est disposé à l'extrémité distale de la gaine de guidage (5) et qui n'est pas transpercé par la canule (3) dans la position de repos de l'unité pour aiguille (12), et est transpercé par la canule (3) dans la position active de l'unité pour aiguille (12), **caractérisée en ce que** le poussoir (2) comporte au moins un ergot de guidage (1) qui peut être amené en prise au choix avec l'une des deux voies de coulissement (10) prévues dans la gaine de guidage (5), lesdites voies de coulissement (10) ayant des pentes opposées.

2. Seringue d'injection (20) selon la revendication 1, **caractérisée en ce que** le poussoir (2) comporte deux ergots de guidage (1) qui sont disposés à distance l'un de l'autre dans un plan perpendiculaire à l'axe longitudinal et dans le sens périphérique, et en vue du déplacement de l'unité pour aiguille (12) depuis la position de repos vers la position active, un seul des deux ergots de guidage (1) peut être amené en prise avec l'une des deux voies de coulissement (10).

3. Seringue d'injection (20) selon la revendication 2, **caractérisée en ce qu'**un dégagement (18) est réalisé dans la gaine de guidage (5) dans une zone périphérique entre les deux voies de coulissement (10), et ledit dégagement (18) est destiné à recevoir l'ergot de guidage (1) qui n'est pas en prise avec les voies de coulissement (10) pendant le déplacement de l'unité pour aiguille (12).

4. Seringue d'injection (20) selon l'une quelconque des revendications 1 à 3, **caractérisée en ce que** l'unité pour aiguille (12), en particulier son porte-canule (4), et la gaine de guidage (5) dans la position active sont maintenues en position l'une par rapport à l'autre dans le sens axial au moyen d'éléments d'arrêt (33, 34) coopérants d'un dispositif d'arrêt (35).

5. Seringue d'injection (20) selon l'une quelconque des revendications 1 à 4, **caractérisée en ce que** l'unité pour aiguille (12), en particulier son porte-canule (4), est couplée au poussoir (2) de manière mobile en rotation autour de l'axe longitudinal, mais immobile dans le sens axial.

6. Seringue d'injection (20) selon l'une quelconque des revendications 1 à 5, **caractérisée en ce que** l'unité pour aiguille (12), en particulier son porte-canule (4), est logée mobile en ligne droite dans le sens axial dans la gaine de guidage (5).

7. Seringue d'injection (20) selon l'une quelconque des revendications 1 à 6, **caractérisée en ce qu'**à l'extrémité de chaque voie de coulissement (10) est prévu un élargissement de coulisse (11), dans lequel l'ergot de guidage (1) peut s'engager dans la position active de l'unité pour aiguille (12).

8. Seringue d'injection (20) selon l'une quelconque des revendications 1 à 7, **caractérisée en ce que** l'extrémité distale de la gaine de guidage (5) est réalisée sous la forme d'un raccord (6).

9. Seringue d'injection (20) selon l'une quelconque des revendications 1 à 8, **caractérisée en ce que** sur la face extérieure de la gaine de guidage (5) est disposée au moins une nervure longitudinale (31), qui prend appui de manière solidaire en rotation sur un organe de blocage (32) d'un capuchon de protection (14) recevant la tête de seringue (21).

10. Seringue d'injection (20) selon la revendication 1, **caractérisée en ce que** l'unité d'injection (25) comporte une tige de piston (17), un élément d'accouplement (37) avec un corps de base (40) et une saillie d'accouplement (41) saillante sur la face détournée de la tige de piston (17), et un bouchon de piston (16), le corps de base (40) de l'élément d'accouplement (37) étant couplé de manière amovible à la tige de piston (17), et le bouchon de piston (16) comportant un trou borgne (38) qui s'étend dans celui-ci à partir d'une extrémité orientée vers la tige de piston (17) et qui est fermé par un fond (39), la saillie d'accouplement (41) de l'élément d'accouplement (37) s'engageant dans le trou borgne (38) et, dans une position initiale inactive du bouchon de piston (16), étant située à distance du corps de base (40) dans le sens axial et, en outre, le fond (39) du trou borgne (38) n'étant pas transpercé par l'extrémité (42) de la saillie d'accouplement (41), au moins un premier élément de blocage (43) étant disposé sur la saillie d'accouplement (41), lequel, dans la position initiale inactive, est inséré dans un évidement de blocage (44) disposé dans le bouchon de piston (16) dans la zone du trou borgne (38).

11. Seringue d'injection (20) selon la revendication 10, **caractérisée en ce que**, lorsque le corps de base (40) et le bouchon de piston (16) sont en contact l'un avec l'autre sur le fond (39) du trou borgne (38), le premier élément de blocage (43) est en appui dans la zone transpercée par la saillie d'accouplement (41).

12. Seringue d'injection (20) selon la revendication 10 ou 11, **caractérisée en ce que** sur la saillie d'accouplement (41), entre le corps de base (40) et le premier élément de blocage (43), est disposé un autre élément de blocage (48) qui, lors du mouvement de rappel de l'unité d'injection (25), prend appui contre un épaulement (49) réalisé dans le trou borgne (38) du bouchon de piston (16).

13. Seringue d'injection (20) selon l'une quelconque des revendications 10 à 12, **caractérisée en ce que** l'extrémité (42) de la saillie d'accouplement est réalisée en se rétrécissant en forme de flèche vers le côté détourné de la tige de piston (17) et comporte au moins un, de préférence deux bras de blocage (45) s'avançant au-delà de la section transversale de la saillie d'accouplement (41).

14. Seringue d'injection (20) selon l'une quelconque des revendications 10 à 13, **caractérisée en ce que** le corps de base (40) de l'élément d'accouplement (37), sur le côté orienté vers la tige de piston (17), comporte des bras d'accouplement (46) élastiquement déformables qui sont couplés de manière amovible à un élément d'accouplement (47) réalisé sur la tige de piston (17).

15. Seringue d'injection (20) selon la revendication 14, **caractérisée en ce que** celle-ci comporte, en plus, un élément de manoeuvre (52) qui peut être couplé à une extrémité distale d'un cylindre d'injection (15), et une largeur intérieure (54) d'un canal de passage (55) dans l'élément de manoeuvre (52) est supérieure dans une zone distale à une section transversale extérieure des bras d'accouplement (46) dans leur position non déformée.

16. Seringue d'injection (20) selon la revendication 15, **caractérisée en ce qu'**entre le corps de base (40) de l'élément d'accouplement (37) et l'élément de manoeuvre (52) est réalisé un dispositif de retenue (56) avec des éléments de retenue (57, 58) coopérants qui, lorsque les éléments de retenue (57, 58) sont en prise, empêchent au moins un déplacement axial de l'élément d'accouplement (37) dans la direction proximale.

17. Seringue d'injection (20) selon la revendication 15 ou 16, **caractérisée en ce que** sur l'élément de manoeuvre (52), dans la zone de son extrémité distale, est disposé au moins un élément conducteur (58) qui s'engage à l'intérieur de la section transversale du canal de passage (55).

18. Seringue d'injection (20) selon l'une quelconque des revendications 10 à 17, **caractérisée en ce qu'**entre l'élément de manoeuvre (52) et la tige de piston (17) est prévu un dispositif de rappel (64), par lequel la tige de piston (17), l'élément d'accouplement (37), le bouchon de piston (16), ainsi que l'unité pour aiguille (12), pouvant être couplée à celui si nécessaire, peuvent être déplacés en retour dans la direction distale par rapport au cylindre d'injection (15).

19. Seringue d'injection (20) selon la revendication 18, **caractérisée en ce que** le dispositif de rappel (64) comporte au moins un ressort (65) qui est en appui avec son extrémité proximale sur l'élément de manoeuvre (52) et avec son extrémité distale sur un coulisseau (66) tubulaire, et, dans la position précontrainte du ressort (65), ledit coulisseau (66) est maintenu, de manière amovible si nécessaire, sur l'élément de manoeuvre (52) au moyen d'un dispositif de retenue (67).

20. Seringue d'injection (20) selon la revendication 19, **caractérisée en ce que**, après un trajet de déplacement prédéterminé de la tige de piston (17) par rapport au cylindre d'injection (15), le dispositif de retenue (67) est libéré par des éléments de réglage (70, 71) coopérants, au moins un premier élément de réglage (70) étant disposé sur le coulisseau (66) et au moins un autre élément de réglage (71) étant disposé sur la tige de piston (17).

21. Seringue d'injection (20) selon l'une quelconque des revendications précédentes, **caractérisée en ce que** le matériau du cylindre d'injection (15) est le verre, et un médicament est introduit dans le volume intérieur de la seringue dans l'attente d'être administré, le volume intérieur de la seringue recevant le médicament est totalement rendu étanche aux bactéries par rapport à la paroi intérieure du cylindre d'injection (15) au niveau de son extrémité proximale par l'élément d'étanchéité (7) non transpercé jusqu'au moment de l'activation et au niveau de son extrémité distale par le bouchon de piston (16) également non transpercé.
